# EUROPEAN PATENT APPLICATION

(11) **EP 4 755 917 A1**
(43) Date of publication of application: **10.06.2026**
(21) Application number: 24850935.8
(22) Date of filing: 02.08.2024
(51) Int. Cl.: C07K 16/28, C12N 15/13, A61K 39/395, A61P 35/00

(54) **ANTI-OX40L ANTIBODY AND USE THEREOF**

(30) Priority: 04.08.2023 CN 202310976269; 10.08.2023 CN 202311004246
(71) Applicant: Fortvita Biologics Inc., 1205 Grand Cayman (KY)
(72) Inventor: RAN, Hao, Suzhou, Jiangsu 215123 (CN); LI, Li, Suzhou, Jiangsu 215123 (CN); HE, Fufan, Suzhou, Jiangsu 215123 (CN); CAO, Lei, Suzhou, Jiangsu 215123 (CN); ZHANG, Yue, Suzhou, Jiangsu 215123 (CN)
(74) Representative: Sagittarius IP
(86) International application number: PCT/CN2024/109523
(87) International publication number: WO 2025/031290

(57) **Abstract**

The present invention relates to novel antibodies and antibody-binding fragments that specifically bind to OX40L and compositions containing the antibodies or antibody-binding fragments. In addition, the present invention relates to nucleic acids encoding the antibodies or antibody-binding fragments thereof and host cells containing the same, as well as related uses. In addition, the present invention relates to therapeutic and diagnostic uses of these antibodies and antibody -binding fragments.

## Description

### Technical field

The present invention relates to novel antibodies and antibody-binding fragments that specifically bind to OX40L and compositions containing the antibodies or antibody fragments. In addition, the present invention relates to nucleic acids encoding the antibodies or antibody-binding fragments thereof and host cells containing the same, as well as related uses. In addition, the present invention relates to therapeutic and diagnostic uses of these antibodies and antibody-binding fragments.

### Background technology

T cell costimulatory molecule OX40 and its homologous ligand OX40L have attracted extensive research interest as therapeutic targets for T cell-mediated diseases.

OX40 is a type I transmembrane glycoprotein, consists of approximately 275 amino acids, having three complete cysteine-rich domains (CRD) and a partial C-terminal CRD. The apparent molecular weight of OX40 is 50 kDa. OX40, along with other TNFRSF members (e.g., 4-1BB, CD27, CD30, and CD40), are T cell costimulatory molecules that play roles at different stages to regulate and control immune responses. Unlike other constitutively expressed T cell costimulatory receptors (such as CD28 and CD27), OX40 is not expressed on naive T cells. OX40 is transiently induced by the signal following TCR engagement following antigen (Ag) recognition, peaking at 48-72 hours in activated T cells (including activated CD4+ and CD8+ T cells, as well as neutrophils, natural killer cells (NK), and natural killer T cells (NKT)). The only exception is that OX40 is constitutively expressed in mouse FOXP3+ Treg cells. The kinetics of OX40 expression involve many factors, including the cytokine environment, antigen persistence, inflammatory context, and the influence of other costimulatory pathways. OX40-deficient T cells usually proliferate and differentiate into effector T cells 2-3 days after TCR engagement, but they show a significant decline in survival at 12-13 days. That is to say, OX40 signaling may not affect the initial activation phase of T cells, but may play an important role in maintaining the late proliferation and survival of T cells during the effector phase.

The ligand of OX40 (OX40L, also known as CD252, CD134L, or gp34) is also a member of the TNFR superfamily. OX40L is a type II glycoprotein, with a 23-amino acid cytoplasmic tail and a 133-amino acid extracellular domain. Mouse OX40L can stimulate both human and mouse T cells; however, human OX40L can only stimulate human T cells. OX40L is mainly expressed on activated antigen-presenting cells (APCs), such as dendritic cells (DCs), activated B cells, and macrophages. The cell types that can be induced to express OX40L are much more extensive. In addition to APCs, OX40L is also expressed on hematopoietic cells, such as activated NK cells, mast cells, or responsive CD4+ T cells, and non-hematopoietic cells, such as endothelial cells or smooth muscle cells (Yu Fu et al., The significance of OX40 and OX40L to T-cell biology and immune disease, Immunological Reviews 2009, Vol. 229: 173-191). OX40L interacts with OX40 and induces receptor trimerization, thereby activating the downstream NF-κB signaling pathway, promoting the formation of memory T cells, the secretion of inflammatory cytokines, and inhibiting the generation of regulatory T cells, which serves to enhance cellular immune responses.

A typical characteristic of autoimmune diseases is the production of reactive autoantibodies against self-antigens due to the immune system's failure to maintain self-tolerance to self-antigens. Due to the limited understanding of the pathogenesis, the incidence of some well-known autoimmune diseases is quite high. These include experimental autoimmune encephalomyelitis (EAE), systemic lupus erythematosus (SLE), and rheumatoid arthritis (RA), colitis, autoimmune experimental uveitis (AEU), type 1 diabetes, and multiple sclerosis (MS).

Given the key role of T cells in controlling the immune response and costimulatory function of OX40-OX40L interaction with T cells, targeting the OX40-OX40L interaction may improve self-antigen-specific T cell responses. Therefore, experiments have been conducted to verify whether targeting the OX40-OX40L interaction by blocking OX40 or OX40L has therapeutic benefits for autoimmune diseases, including EAE, SLE, RA, colitis, AEU, type 1 diabetes, MS, graft-versus-host disease (GVHD), and inflammatory bowel disease (IBD). Studies have shown that OX40 is upregulated at autoimmune sites and is associated with disease severity. Thus, inhibiting this signaling pathway has the potential for therapeutic effects on various immune-related diseases.

Due to the large number of unmet clinical treatment needs, it is necessary to develop drugs targeting OX40L. Although some monoclonal antibodies against OX40L have been developed using existing technologies, these antibodies have low affinity, and their biological activity and in vivo efficacy need to be further improved.

Therefore, there is still a need to develop new antibodies against OX40L that have stronger binding affinity to OX40L, higher biological activity, and better in vivo efficacy.

### Summary of the Invention

The invention provides a new high-affinity antibody that recognizes OX40 ligand (OX40L) which selectively blocks the OX40L-OX40 signaling pathway.

Thus, the invention relates to an antibody or fragment, such as an antigen-binding fragment, that specifically binds to OX40L, for example, human OX40L.

The antibodies or antigen-binding fragments of the invention can effectively block the interaction between OX40L and its receptor OX40 at both protein and cellular levels, and can effectively inhibit the activation, proliferation, and/or differentiation of T cells.

For example, in a mouse model of graft-versus-host disease (GvHD), mice administered with the antibodies or antigen-binding fragments of the invention can maintain weight gain and are symptom-free, and/or show significantly reduced T cell activation or production of inflammatory factors.

The antibodies or antigen-binding fragments of the invention, through mutations in the Fc region, such as an S228P mutation, can effectively reduce the likelihood of antibody chain exchange in vivo and enhance their stability.

Therefore, the present invention relates to the following specific aspects.
In some aspects, the present invention relates to an anti-OX40L antibody or an antigen-binding fragment thereof, the antibody comprising:
(i) three complementarity-determining regions HCDR1, HCDR2, and HCDR3 contained in the VH as set forth in SEQ ID NO:4, and the three complementarity-determining regions LCDR1, LCDR2, and LCDR3 contained in the VL as set forth in SEQ ID NO:8; or
(ii) the three complementarity-determining regions HCDR1, HCDR2, and HCDR3 contained in the VH as set forth in SEQ ID NO: 14, and the three complementarity-determining regions LCDR1, LCDR2, and LCDR3 contained in the VL as set forth in SEQ ID NO:18;
for example, wherein the HCDR1 is determined by AbM scheme, HCDR2, HCDR3, LCDR1, LCDR2 and LCDR3 is deteremined according to Kabat scheme.

In some aspects, the present invention relates to an anti-OX40L antibody or an antigen-binding fragment thereof, comprising a first heavy chain complementary determining region (HCDR1), a second heavy chain complementary determining region (HCDR2), a third heavy chain complementary determining region (HCDR3), and a first light chain complementary determining region (LCDR1), a second light chain complementary determining region (LCDR2), and a third light chain complementary determining region (LCDR3), wherein
(i) the HCDR1, HCDR2, HCDR3, and LCDR1, LCDR2, and LCDR3 comprise the amino acid sequences set forth in SEQ ID NO:1, SEQ ID NO:2, SEQ ID NO:3, SEQ ID NO:5, SEQ ID NO:6, and SEQ ID NO:7 respectively, or consist of the amino acid sequences set forth in SEQ ID NO:1, SEQ ID NO:2, SEQ ID NO:3, SEQ ID NO:5, SEQ ID NO:6, and SEQ ID NO:7 respectively; or
(ii) the HCDR1, HCDR2, HCDR3, and LCDR1, LCDR2, and LCDR3 comprise the amino acid sequences set forth in SEQ ID NO:11, SEQ ID NO:12, SEQ ID NO:13, SEQ ID NO:15, SEQ ID NO:16, and SEQ ID NO:17 respectively, or consist of the amino acid sequences set forth in SEQ ID NO:11, SEQ ID NO:12, SEQ ID NO:13, SEQ ID NO:15, SEQ ID NO:16, and SEQ ID NO:17 respectively.

In some embodimens, the anti-OX40L antibody or antigen-binding fragment of the present invention comprises a heavy chain variable region (VH), wherein said heavy chain variable region comprises an amino acid sequence having at least 90% identity to the amino acid sequence as set forth in SEQ ID NO:4 or 14, or consists of said sequence or comprises the amino acid sequence as set forth in SEQ ID NO:4 or 14, or consists of said sequence.

In some embodimens, the anti-OX40L antibody or antigen-binding fragment of the present invention comprises a light chain variable region (VL), wherein said light chain variable region comprises an amino acid sequence having at least 90% identity to the amino acid sequence as set forth in SEQ ID NO:8 or 18, or consists of said sequence or comprises the amino acid sequence as set forth in SEQ ID NO:8 or 18, or consists of said sequence.

In some aspects, the present invention relates to an anti-OX40L antibody or an antigen-binding fragment thereof, comprising a heavy chain variable region and a light chain variable region,
wherein
(i) the heavy chain variable region comprises or consists of the amino acid sequence set forth in SEQ ID NO: 4, or an amino acid sequence having at least 90%, 91%, 92%, 93%, 94%, 95%, 96%, 97%, 98% or 99% identity thereto; and the light chain variable region comprises or consists of the amino acid sequence set forth in SEQ ID NO: 8, or an amino acid sequence having at least 90%, 91%, 92%, 93%, 94%, 95%, 96%, 97%, 98% or 99% identity thereto;
(ii) the heavy chain variable region comprises or consists of the amino acid sequence set forth in SEQ ID NO: 14, or an amino acid sequence having at least 90%, 91%, 92%, 93%, 94%, 95%, 96%, 97%, 98% or 99% identity thereto; and the light chain variable region comprises or consists of the amino acid sequence set forth in SEQ ID NO: 18, or an amino acid sequence having at least 90%, 91%, 92%, 93%, 94%, 95%, 96%, 97%, 98% or 99% identity thereto.

In some embodimens, the anti-OX40L antibody or antigen-binding fragment of the present invention comprises a heavy chain variable region and a light chain variable region, wherein
(i) the heavy chain variable region comprises or consists of the amino acid sequence set forth in SEQ ID NO: 4, and the light chain variable region comprises or consists of the amino acid sequence set forth in SEQ ID NO: 8; or
(ii) the heavy chain variable region comprises or consists of the amino acid sequence set forth in SEQ ID NO: 14, and the light chain variable region comprises or consists of the amino acid sequence set forth in SEQ ID NO: 18.

In some embodimens, the anti-OX40L antibody or antigen-binding fragment of the present invention comprises comprises an Fc region. In some specific embodiments, said Fc region is an Fc from a human IgG, for example, an Fc from human IgG1, an Fc from human IgG2, an Fc from human IgG3, or an Fc from human IgG4. In some specific embodiments, said Fc region comprises a mutation that enhances the stability of the Fc region or reduces antibody chain exchange in vivo. In some specific embodiments, said mutation is an S228P mutation. In some specific embodiments,
wherein said Fc region
(i) comprises or consists of the amino acid sequence of SEQ ID NO:27 or 28;
(ii) comprises or consists of an amino acid sequence having at least 90% identity, for example, 95%, 96%, 97%, 99% or higher identity, to the amino acid sequence set forth in SEQ ID NO:28; or
(iii) comprises an amino acid sequence having at least 90% identity, for example, 95%, 96%, 97%, 99% or higher identity, to the amino acid sequence set forth in SEQ ID NO:27 and comprising an S228P mutation.

In some embodimens, the anti-OX40L antibody or antigen-binding fragment of the present invention comprises a heavy chain constant region, said heavy chain constant region being from the constant region of IgG1, IgG2, IgG3 or IgG4, preferably, said heavy chain constant region
(i) comprises or consists of the amino acid sequence selected from SEQ ID NO:21 or 29;
(ii) comprises an amino acid sequence having at least 85%, 90%, 91%, 92%, 93%, 94%, 95%, 96%, 97%, 98% or 99% identity to the amino acid sequence set forth in SEQ ID NO:29; or
(iii) comprises an amino acid sequence having at least 85%, 90%, 91%, 92%, 93%, 94%, 95%, 96%, 97%, 98% or 99% identity to the amino acid sequence set forth in SEQ ID NO:21 and having an S228P deletion mutation.

In some embodimens, the anti-OX40L antibody or antigen-binding fragment of the present invention comprises a light chain constant region, said light chain constant region being a lambda or kappa light chain constant region, preferably, said light chain constant region
(i) comprises or consists of an amino acid sequence having at least 85%, 90%, 91%, 92%, 93%, 94%, 95%, 96%, 97%, 98% or 99% identity to the amino acid sequence of SEQ ID NO:22 said amino acid sequence; or
(ii) comprises or consists of the amino acid sequence of SEQ ID NO:22.

In some embodimens, the anti-OX40L antibody or antigen-binding fragment of the present invention comprises a heavy chain, wherein said heavy chain
(i) comprises an amino acid sequence having at least 85%, 90%, 91%, 92%, 93%, 94%, 95%, 96%, 97%, 98% or 99% identity with an amino acid sequence selected from SEQ ID NO: 9 or 19 or consists of the amino acid sequence; or
(ii) comprises an amino acid sequence selected from SEQ ID NO: 9 or 19 or consists of the amino acid sequence.

In some embodimens, the anti-OX40L antibody or antigen-binding fragment of the present invention comprises a light chain, wherein said light chain
(i) comprises an amino acid sequence having at least 85%, 90%, 91%, 92%, 93%, 94%, 95%, 96%, 97%, 98% or 99% identity with an amino acid sequence selected from SEQ ID NO: 10 or 20 or consists of the amino acid sequence; or
(ii) comprises an amino acid sequence selected from SEQ ID NO: 10 or 20 or consists of the amino acid sequence.

In some embodimens, the present invention relates to the anti-OX40L antibody or antigen-binding fragment comprising a heavy chain and a light chain, wherein
(i) the heavy chain comprises or consists of an amino acid sequence having at least 85%, 90%, 91%, 92%, 93%, 94%, 95%, 96%, 97%, 98% or 99% identity with the amino acid sequence set forth in SEQ ID NO:9, and the light chain comprises or consists of an amino acid sequence having at least 85%, 90%, 91%, 92%, 93%, 94%, 95%, 96%, 97%, 98% or 99% identity with the amino acid sequence set forth in SEQ ID NO:10; or
(ii) the heavy chain comprises or consists of an amino acid sequence having at least 85%, 90%, 91%, 92%, 93%, 94%, 95%, 96%, 97%, 98% or 99% identity with the amino acid sequence set forth in SEQ ID NO:19, and the light chain comprises or consists of an amino acid sequence having at least 85%, 90%, 91%, 92%, 93%, 94%, 95%, 96%, 97%, 98% or 99% identity with the amino acid sequence set forth in SEQ ID NO:20.

In some embodimens, the anti-OX40L antibody or antigen-binding fragment of the present invention comprises a heavy chain and a light chain, wherein
(i) the heavy chain comprises or consists of the amino acid sequence of SEQ ID NO:9, and the light chain comprises or consists of the amino acid sequence of SEQ ID NO:10; or
(ii) the heavy chain comprises or consists of the amino acid sequence of SEQ ID NO: 19, and the light chain comprises or consists of the amino acid sequence of SEQ ID NO:20.

In some embodimens, the anti-OX40L antibody of the present invention is a monoclonal antibody.

In some embodimens, the anti-OX40L antibody of the present invention is a humanized antibody or a chimeric antibody.

In some embodimens, the antigen-binding fragment of the anti-OX40L antibody of the present invention is an antibody fragment selected from the group consisting of Fab, Fab', Fab'-SH, Fv, single-chain antibody (e.g., scFv), (Fab')₂, single-domain antibody such as VHH, dAb (domain antibody), bivalent antibody, or linear antibody.

In some embodimens, the anti-OX40L antibody or antigen-binding fragment of the present invention has one or more of the following properties:
a) binds to OX40L (e.g., human OX40L) with high affinity;
b) effectively blocks the interaction between OX40L and its receptor OX40 at both protein and cellular levels;
c) inhibits the OX40/OX40L signaling pathway;
d) inhibits the activation, proliferation, and/or differentiation of T cells;
e) reduces the activation of T cells and/or the production of inflammatory factors;
f) treats graft-versus-host disease.

In some embodiments, the present invention relates to an isolated nucleic acid encoding the anti-OX40L antibody or antigen-binding fragment of the present invention.

In some embodiments, the present invention relates to a vector comprising the nucleic acid of the present invention, preferably said vector is an expression vector.

In some embodiments, the present invention relates to a host cell comprising the nucleic acid or the vector of the present invention, preferably said host cell is prokaryotic or eukaryotic, more preferably selected from yeast cells, mammalian cells (e.g., 293 cells or CHO cells, e.g., CHO-K cells or HEK293 cells), or other cells suitable for the preparation of antibodies or antigen-binding fragments.

In some aspects, the pesent invention relates to a method for preparing an anti-OX40L antibody or an antigen-binding fragment thereof, said method comprising
a) culturing the host cell of the present invention 2 under conditions suitable for the expression of the nucleic acid encoding the anti-OX40L antibody or antigen-binding fragment thereof of the present invention,
b) optionally isolating said antibody or antigen-binding fragment thereof,
c) optionally said method also includes recovering said anti-OX40L antibody or antigen-binding fragment thereof from said host cell, optionally, said antibody is purified, e.g., by Protein A purification.

In some aspects, the pesent invention relates to an immunoconjugate comprising the anti-OX40L antibody or antigen-binding fragment of the present invention and another agent, such as a toxin, small molecule drug, cytotoxic agent, cellular apoptotic agent, chelating agent, immune modulator, such as an anti-inflammatory agent or immunosuppressive agent.

In some aspects, the pesent invention relates to a pharmaceutical composition comprising the anti-OX40L antibody or antigen-binding fragment or the immunoconjugate of the present invention, and optionally a pharmaceutically acceptable excipient.

In some aspects, the pesent invention relates to a pharmaceutical combination comprising the anti-OX40L antibody or antigen-binding fragment or the immunoconjugate of the present invention, and one or more other therapeutic agents, such as said therapeutic agent is selected from the group consisting of cytokines, other antibodies, small molecule drugs, or immune modulators (e.g., immunosuppressive agents).

In some aspects, the pesent invention relates to a method for preventing or treating a disease or condition that are associated with abnormal activation of OX40L/OX40-mediated pathways in an individual, said method comprising administering to said subject an effective amount of the anti-OX40L antibody or antigen-binding fragment, or the immunoconjugate, or the pharmaceutical composition, or the pharmaceutical combination of the present invention. In some embodiments, said subject has abnormal activation of the OX40L/OX40-mediated signaling pathway compared to a healthy individual. In some embodiments, said disease or condition is selected from autoimmune diseases or graft-versus-host disease, for example, the autoimmune diseases include, but are not limited to, allergic dermatitis, asthma, systemic lupus erythematosus, Sjogren's syndrome, immune thrombocytopenic purpura, multiple sclerosis, lupus nephritis, amyotrophic lateral sclerosis, rheumatoid arthritis, non-rheumatoid arthritis, contact dermatitis, hyper IgE syndrome, inflammatory bowel disease, myasthenia gravis, Graves' disease, hemolytic anemia, psoriasis, atopic dermatitis, allergic asthma, or idiopathic inflammatory disease. In some embodiments, said method further comprises administering one or more other therapies, such as therapeutic modalities and/or other therapeutic agents, such as said therapeutic agent is selected from the group consisting of cytokines, other antibodies, small molecule drugs, or immune modulators (e.g., immunosuppressive agents).

### Figures

Figure 1 shows the affinity of anti-OX40L antibodies, their blocking activity against the OX40-OX40L interaction, and the effect on luciferase activity in Jurkat cells overexpressing OX40.
Figure 2 shows the effect of anti-OX40L antibodies on T cell activation.
Figure 3 shows the changes in IL-5 and IL-13 secretion after treatment with anti-OX40L antibodies during DC-induced Th2 differentiation.
Figure 4 shows the changes in mouse body weight and survival curves in a GvHD mouse model.

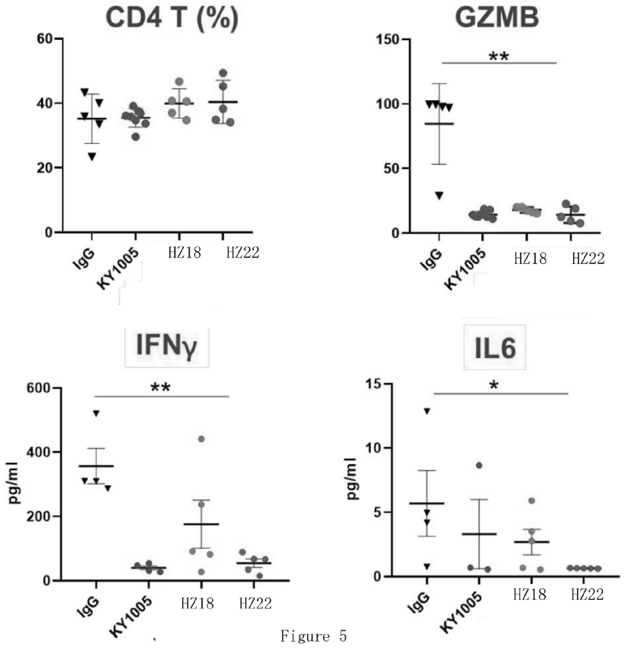

Figure 5 shows the proportion of CD4-positive T cells in the blood of mice on day 14 in a GvHD mouse model.

### Detailed description of the invention

### I. Definitions

Before describing the present invention in detail below, it should be understood that the present invention is not limited to the specific methodology, protocols and agents described herein, as these may vary. It should be understood that the terms used herein are only for the purpose of describing specific embodiments and are not intended to limit the scope of the present invention, which will be limited only by the appended claims. Unless otherwise defined, all technical and scientific terms used herein have the same meaning as commonly understood by one of ordinary skill in the art to which the present invention belongs.

For the purpose of interpreting this specification, the following definitions will be used, and whenever appropriate, terms used in the singular may also include the plural, and vice versa. It is to be understood that the terms used herein are only for the purpose of describing specific embodiments and are not meant to limit.

The term "about" when used in conjunction with a numerical value is intended to encompass numerical values within a range having a lower limit of 5% less than the specified numerical value and an upper limit of 5% greater than the specified numerical value.

As used herein, the term "and/or" means any one of the optional items or two or more or all of the optional items.

As used herein, the term "comprise" or "include" means including the elements, integers or steps described, but not excluding any other elements, integers or steps. In this context, when the term "comprise" or "include" is used, unless otherwise specified, the situation consisting of the elements, integers or steps described is also covered. For example, when referring to an antibody variable region "comprising" a specific sequence, it is also intended to cover the antibody variable region consisting of the specific sequence.

An "isolated" antibody or molecule is an antibody or molecule that has been separated from a component of its natural environment. In some embodiments, the antibody or molecule is purified to more than 95% or 99% purity, as determined by, for example, electrophoresis (e.g., SDS-PAGE, isoelectric focusing (IEF), capillary electrophoresis) or chromatography (e.g., ion exchange or reverse phase HPLC).

As used herein, "OX40L" refers to any native OX40L polypeptide (e.g., human OX40L polypeptide) or its variants. The term "OX40L" covers the "full-length" unprocessed OX40L polypeptide as well as any form of OX40L polypeptide produced by intracellular processing. The term also encompasses naturally occurring variants of OX40L, such as those encoded by splice variants and allelic variants. The OX40L polypeptides described herein may be obtained from a variety of sources, such as from human tissue or from another source, for example, cynomolgus monkey, or may be prepared by recombinant or synthetic methods. In one embodiment of the invention, the human OX40L protein is as shown in (CD252, UniProt: P23510). In one embodiment, the human OX40L gene is as shown in (UniProt: P29279). In one embodiment of the invention, the cynomolgus monkey OX40L protein is as shown in (UniProt: A0A1D5QQH7).

The terms "whole antibody" or "full-length antibody" are used interchangeably herein and refer to an antibody molecule having the structure of natural immunoglobulin molecules. In the case of conventional four-chain IgG antibodies, the full-length antibody comprises two heavy chains (H) and two light chains (L) interconnected by a disulfide bond. In the case of a heavy chain antibody having only a heavy chain and lacking a light chain, the full-length antibody comprises two heavy chains (H) interconnected by a disulfide bond. For a conventional four-chain IgG antibody, the full-length antibody heavy chain is generally consists of a heavy chain variable region (abbreviated herein as VH) and a heavy chain constant region, wherein the heavy chain constant region comprises at least three domains CH1, CH2 and CH3. The full-length antibody light chain consists of a light chain variable region (abbreviated herein as VL) and a light chain constant region, wherein the light chain constant region consists of one domain CL. Each heavy chain variable region VH and each light chain variable region VL consists of three CDRs and four FRs, arranged from the amino terminus to the carboxyl terminus in the following order: FR1, CDR1, FR2, CDR2, FR3, CDR3, FR4. The term "antibody fragment" includes a portion of a complete antibody. In a preferred embodiment, the antibody fragment is an antigen binding fragment.

The term "antigen binding fragment" of an antibody is a molecule different from a full-length antibody, which contains a portion of a full-length antibody, but which can bind to the antigen of the full-length antibody or compete with the full-length antibody (i.e., the full-length antibody from which the antigen binding fragment is derived) for binding to the antigen. Antigen binding fragments can be prepared by recombinant DNA technology, or by enzymatic or chemical cleavage of complete antibodies. Antigen binding fragments include, but are not limited to, Fv, Fab, Fab', Fab'-SH, F(ab')2, dAb (domain antibody), linear antibodies, single-chain antibodies (e.g., scFv); single-domain antibodies such as VHH, diabodies or fragments thereof, or camelid antibodies, diabodies, single-domain antibodies (sdAb), nanobodies. For example, Fab fragments can be obtained by digesting full-length antibodies with papain. In addition, digesting full antibodies with pepsin below the disulfide bonds in the hinge region produces F(ab')2, which is a dimer of Fab' and is a divalent antibody fragment. F(ab')2 can be reduced under neutral conditions by breaking the disulfide bonds in the hinge region, thereby converting the F(ab')2 dimer into a Fab' monomer. The Fab' monomer is basically a Fab fragment with a hinge region. The Fv fragment consists of the VL and VH domains of a single arm of an antibody. The two domains of the Fv fragment, VL and VH, can be encoded by separate genes, but recombinant methods can also be used to connect the two domains using synthetic connecting peptides to produce them as a single protein chain, and in the single protein chain, the VL region and the VH region are paired to form a single-chain Fv (scFv).

The term "single-chain antibody (scAb)" is used in the broadest sense herein and specifically covers antibodies with monospecificity or multispecificity (e.g., bispecificity) that were originally produced as a single continuous polypeptide chain. Such single-chain antibodies include, but are not limited to, having two linked VL and VH regions. In one embodiment, the single-chain antibody is an scFv.

"Diabody" is a small bivalent antibody constructed by gene fusion, for example, a dimer consisting of two polypeptide chains. The VL and VH domains of each polypeptide chain of the diabody are connected by a linker, so that the VL and VH encoded in the same polypeptide chain form a dimer with different single-chain variable region fragments. Diabodies generally have two antigen binding sites.

"Complementarity determining region" or "CDR region" or "CDR" is a region in the variable domain of an antibody that is highly variable in sequence and forms a structurally defined loop ("hypervariable loop") and/or contains antigen contact residues ("antigen contact point"). CDR is primarily responsible for binding to an antigen epitope. The CDRs of the heavy and light chains are usually referred to as CDR1, CDR2, and CDR3, numbered sequentially from the N-terminus. The CDRs located within the antibody heavy chain variable domain are called HCDR1, HCDR2, and HCDR3, while the CDRs located within the antibody light chain variable domain are called LCDR1, LCDR2, and LCDR3. In a given light chain variable region or heavy chain variable region amino acid sequence, the precise amino acid sequence boundaries of each CDR can be determined using any one or a combination of a number of well-known antibody CDR assignment schemes, including, for example, Chothia (Chothia et al. (1989) Nature 342: 877-883, Al-Lazikani et al., "Standard conformations for the canonical structures of immunoglobulins", Journal of Molecular Biology, 273, 927-948 (1997)), Kabat based on the variability of antibody sequences (Kabat et al., Sequences of Proteins of Immunological Interest, 4th Edition, U.S. Department of Health and Human Services, National Institutes of Health (1987)), AbM (University of Bath), Contact (University College London), the international ImMunoGeneTics database (IMGT) (http://imgt.cines.fr or http://imgt.cines.org), and the definition of the North CDR based on affinity propagation clustering using a large number of crystal structures.

Below are exemplary schemes for the region range of CDRs defined using the Kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering system) | L24-L34 | L24-L34 | L26-L32 | L30-L36 | L27-L32 |
| LCDR2 (Kabat and Chothia Chothia numbering system) | L50-L56 | L50-L56 | L50-L52 | L46-L55 | L50-L52 |
| LCDR3 (Kabat and Chothia Chothia numbering system) | L89-L97 | L89-L97 | L91-L96 | L89-L96 | L89-L96 |
| HCDR1 ( Kabat numbering system) | H31-H35B | H26-H35B | H26-H32 | H30-H35B | H26-H35B |
| HCDR1 ( Chothia numbering system) | H31-H35 | H26-H35 | H26-H32 | H30-H35 | H26-H35 |
| HCDR2 (Kabat and Chothia Chothia numbering system) | H50-H65 | H50-H58 | H53-H55 | H47-H58 | H51-H57 |
| HCDR3 (Kabat and Chothia Chothia numbering system) | H95-H102 | H95-H102 | H96-H101 | H93-H101 | H93-H102 |

Unless otherwise specified, in the present invention, the term "CDR" or "CDR sequence" encompasses CDR sequences determined by any schems above. CDRs can also be determined based on the same Kabat numbering position as a reference CDR sequence (e.g., any of the exemplary CDRs of the present invention).

Unless otherwise specified, in the present invention, when referring to residue positions in the variable region of an antibody (including heavy chain variable region residues and light chain variable region residues), it refers to the numbering positions according to the Kabat numbering system (Kabat et al., Sequences of Proteins of Immunological Interest, 5th Ed. Public Health Service, National Institutes of Health, Bethesda, Md. (1991)).

In some embodiments, CDR1 of the heavy chain variable region of an antibody in the present invention is determined according to AbM scheme, CDR2, and CDR3 of the heavy chain variable region of an antibody in the present invention are determined according to the Kabat scheme, respectively. In some embodiments, CDRs of the light chain variable region of an antibody in the present invention are determined according to the Kabat scheme.

An antibody that "binds to the same or overlapping epitope" as a reference antibody refers to an antibody that blocks 50%, 60%, 70%, 80%, 90% or 95% or more of the binding of the reference antibody to its antigen in a competition assay. Conversely, the reference antibody blocks 50%, 60%, 70%, 80%, 90% or 95% or more of the binding of the antibody to its antigen in a competition assay.

An antibody that competes with a reference antibody for binding to its antigen refers to an antibody that blocks 50%, 60%, 70%, 80%, 90% or more of the binding of the reference antibody to its antigen in a competition assay. Conversely, the reference antibody blocks 50%, 60%, 70%, 80%, 90% or more of the binding of the antibody to its antigen in a competition assay. Numerous types of competitive binding assays can be used to determine whether one antibody competes with another, such as solid phase direct or indirect radioimmunoassays (RIA), solid phase direct or indirect enzyme immunoassays (EIA), sandwich competition assays.

An antibody that inhibits (e.g., competitively inhibits) the binding of a reference antibody to its antigen is an antibody that inhibits 50%, 60%, 70%, 80%, 90%, or 95% or more of the binding of the reference antibody to its antigen. Conversely, the reference antibody inhibits 50%, 60%, 70%, 80%, 90%, or 95% or more of the binding of the antibody to its antigen. The binding of an antibody to its antigen can be measured by affinity (e.g., equilibrium dissociation constant). Methods for determining affinity are known in the art.

An antibody that exhibits the same or similar binding affinity and/or specificity as a reference antibody is an antibody that is capable of having at least 50%, 60%, 70%, 80%, 90%, or 95% or more of the binding affinity and/or specificity of the reference antibody. This can be determined by any method known in the art for determining binding affinity and/or specificity.

The term "chimeric antibody" refers to an antibody molecule in which (a) the constant region or a portion thereof is changed, substituted, or exchanged, such that the antigen-binding site is linked to a constant region of a different or altered class, effector function, and/or species, or to a completely different molecule that confers new properties on the chimeric antibody (e.g., enzyme, toxin, hormone, growth factor, drug); or (b) the variable region or a portion thereof is changed, substituted, or exchanged with a variable region having different or altered antigen specificity. For example, a mouse antibody can be modified by replacing its constant region with a constant region from a human immunoglobulin. Due to the replacement with the human constant region, the chimeric antibody can retain its specificity in recognizing the antigen, while having reduced immunogenicity in humans compared to the original mouse antibody.

A "humanized antibody" is an antibody that retains the antigen-specific reactivity of a non-human antibody (e.g., a mouse monoclonal antibody), while having lower immunogenicity when administered to humans as a therapeutic agent. This can be achieved, for example, by retaining the non-human antigen-binding site and replacing the remainder of the antibody with its human counterparts (i.e., replacing the constant regions and portions of the variable regions that do not participate in binding with the corresponding parts of a human antibody).

The term "Fc domain" or "Fc region" is used herein to define the C-terminal region of an immunoglobulin heavy chain that contains at least a portion of the constant region. This term includes both native sequence Fc regions and variant Fc regions. The native immunoglobulin "Fc domain" comprises two or three constant domains, namely the CH2 domain, the CH3 domain, and optionally the CH4 domain. For instance, in native antibodies, the immunoglobulin Fc domain includes the second and third constant domains (CH2 domain and CH3 domain) from the two heavy chains of IgG, IgA, and IgD class antibodies; or it includes the second, third, and fourth constant domains (CH2 domain, CH3 domain, and CH4 domain) from the two heavy chains of IgM and IgE class antibodies. Unless otherwise specified herein, the amino acid residue numbering in the Fc region or heavy chain constant region is according to the EU numbering system (also known as the EU index) as described by Kabat et al., Sequences of Proteins of Immunological Interest, 5th ed., Public Health Service, National Institutes of Health, Bethesda, MD, 1991.

"Conservative changes" as described herein include replacements, deletions or additions to a polypeptide sequence, but do not substantially change the desired functional activity of the polypeptide sequence. In some embodiments, conservative changes are conservative substitutions. Conservative substitutions refer to the replacement of an amino acid with another amino acid within the same class, such as an acidic amino acid replaced by another acidic amino acid, a basic amino acid replaced by another basic amino acid, or a neutral amino acid replaced by another neutral amino acid. For example, conservative substitutions often result in a certain amino acid being replaced with a chemically similar amino acid. Conservative substitution tables providing functionally similar amino acids are well known in the art. The following lists 8 groups of amino acids containing conservative substitutions: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M). In some embodiments, the term "conservative change" is used to refer to an amino acid modification that does not significantly affect or change the target antigen binding characteristics of the antibody molecule of the present invention containing the amino acid sequence when applied to the antibody molecule amino acid sequence. For example, the conservatively changed variant maintains at least 80%, 85%, 90%, 95%, 98%, 99% or higher, such as 100-110% or higher binding affinity to the target antigen relative to the parent antibody.

The term "vector" as used herein refers to a nucleic acid molecule capable of propagating another nucleic acid to which it is attached. The term includes vectors that are self-replicating nucleic acid structures as well as vectors that are incorporated into the genome of a host cell into which it has been introduced. Some vectors are capable of directing the expression of nucleic acids to which they are operably attached. Such vectors are referred to herein as "expression vectors".

An "immunoconjugate" is an antibody conjugated to one or more other substances (including but not limited to a label).

The term "therapeutic agent" as used herein covers any agent that is effective in preventing or treating diseases associated with inappropriate activation of the OX40/OX40L-mediated pathway, including cytokines, other antibodies, small molecule drugs, or immune modulators (e.g., immunosuppressive agents).

The term "small molecule drug" refers to low molecular weight organic compounds that can modulate biological processes. "Small molecule" is defined as a molecule with a molecular weight of less than 10 kD, typically less than 2 kD, and preferably less than 1 kD. Small molecules include, but are not limited to, inorganic molecules, organic molecules, organic molecules containing inorganic components, molecules containing radioactive atoms, synthetic molecules, peptidomimetics, and antibody mimetics. As therapeutic agents, small molecules can be more cell-permeable than large molecules, less susceptible to degradation, and less likely to elicit an immune response.

The term "immune modulator" as used herein refers to natural or synthetic active agents or drugs that suppress or modulate immune responses. The immune response can be a humoral response or a cellular response. Immune modulators include immunosuppressive agents.

The terms "immunosuppressive agent," "immunosuppressive drug," or "immunosuppressive" as used herein refer to therapeutic agents used in immunosuppressive therapy to suppress or inhibit the activity of the immune system.

The term "effective amount" refers to such an amount or dose of the antibody or fragment or conjugate or composition or combination of the present invention that produces the desired effect in a patient in need of treatment or prevention after being administered to the patient in a single or multiple doses.

A "therapeutically effective amount" refers to an amount effective to achieve the desired therapeutic outcome at the required dosage and for the required period of time. A therapeutically effective amount is also an amount in which any toxic or deleterious effects of the antibody or antibody fragment or its conjugate or composition or combination are outweighed by the therapeutically beneficial effects. A "therapeutically effective amount" preferably inhibits a measurable parameter (e.g., T cell activation) by at least about 20%, more preferably at least about 40%, even more preferably at least about 50%, 60%, or 70% relative to an untreated subject. In some embodiments, the term "therapeutically effective amount" as used herein is intended to define the amount of treatment necessary to treat conditions (such as autoimmune, allergies, inflammation, graft-versus-host disease (GVHD), or anti-transplantation, drug, or rejection reactions) or reduce or eliminate immune responsesin a treatment regimen.

A "prophylactically effective amount" refers to an amount effective to achieve the desired prophylactic outcome at the required dosage and for the required period of time. Typically, because prophylactic doses are used in subjects before or at an earlier stage of the disease, the prophylactically effective amount will be less than the therapeutically effective amount. In some embodiments, the term "prophylactically effective amount" as used herein is intended to define the amount of prevention necessary to prevent the progression and symptoms (such as autoimmune, allergies, inflammation, graft-versus-host disease (GVHD), or anti-transplantation, drug, or rejection reactions) of a condition or disease in a treatment regimen.

The terms "host cell," "host cell line," and "host cell culture" are used interchangeably and refer to cells into which exogenous nucleic acids are introduced, including the progeny of such cells. Host cells include "transformants" and "transformed cells," which include primary transformed cells and progeny derived therefrom, regardless of the number of passages. Progeny may not be completely identical to the parent cell in nucleic acid content, but may contain mutations. Included herein are mutant progeny with the same function or biological activity that were screened or selected in the initially transformed cells.

The term "label" as used herein refers to a compound or composition that is directly or indirectly conjugated or fused to an agent (such as a polynucleotide probe or antibody) and facilitates the detection of the agent to which it is conjugated or fused. The label itself may be detectable (e.g., a radioisotope label or a fluorescent label) or, in the case of an enzymatic label, may catalyze a chemical change in a detectable substrate compound or composition. The term is intended to encompass direct labeling of a probe or antibody by coupling (i.e., physically linking) a detectable substance to the probe or antibody and indirect labeling of the probe or antibody by reaction with another reagent that is directly labeled.

As used herein, the terms "subject" "object" or "individual" are used interchangeably and include mammals. Mammals include, but are not limited to, domestic animals (e.g., cows, sheep, cats, dogs, and horses), primates (e.g., humans and non-human primates such as monkeys), rabbits, and rodents (e.g., mice and rats). In some embodiments, the individual or subject is a human.

As used herein, the term "subject/patient/individual sample" refers to a collection of cells or fluids obtained from a patient or subject. The source of the tissue or cell sample can be solid tissue, such as from a fresh, frozen and/or preserved organ or tissue sample or a biopsy sample or puncture sample; blood or any blood component; body fluids, such as cerebrospinal fluid, amniotic fluid, peritoneal fluid (ascites), or interstitial fluid; cells from any time of pregnancy or development of the subject. Tissue samples may contain compounds that are not naturally intermixed with tissues in nature, such as preservatives, anticoagulants, buffers, fixatives, nutrients, antibiotics, and the like.

"Nucleic acid encoding an anti-OX40L antibody or fragment thereof' refers to one or more nucleic acid molecules encoding an antibody heavy chain or light chain (or fragment thereof, such as a heavy chain variable region or a light chain variable region), including such nucleic acid molecules in a single vector or separate vectors, and such nucleic acid molecules present at one or more locations in a host cell.

"Percent identity (%)" of an amino acid sequence refers to the percentage of amino acid residues in a candidate sequence that are identical to the amino acid residues of a specific amino acid sequence shown in this specification after aligning the candidate sequence with the specific amino acid sequence shown in this specification and, if necessary, introducing gaps to achieve the maximum percentage of sequence identity, and not considering any conservative substitutions as part of the sequence identity. In some embodiments, the present invention contemplates variants of the antibody molecules of the present invention, which have a considerable degree of identity relative to the antibody molecules and sequences thereof specifically disclosed herein, such as an identity of at least 80%, 85%, 90%, 95%, 97%, 98% or 99% or more. The variants may contain conservative changes.

The term "pharmaceutically acceptable excipient" refers to a diluent, adjuvant (e.g., Freund's adjuvant (complete and incomplete)), vehicles, carrier or stabilizer, etc., which is administered together with an active substance.

The term "pharmaceutical composition" refers to a composition that is in a form that allows the biological activity of the active ingredients contained therein to be effective and does not contain additional ingredients that are unacceptably toxic to the subject to which the composition is administered.

As used herein, the term "pharmaceutical combination" or "combination product" refers to a non-fixed combination product or a fixed combination product, including but not limited to a kit, a pharmaceutical composition. The term "non-fixed combination" means that the active ingredients (e.g., (i) an antibody of the present invention, and (ii) another therapeutic agent) are administered to a patient simultaneously, or sequentially without specific time limits or at equal or different time intervals, as separate entities, wherein such administration provides preventive or therapeutically effective levels of two or more active agents in the patient. The term "fixed combination" means that two or more active agents are administered to a patient simultaneously in the form of a single entity. The dosages and/or time intervals of the two or more active agents are preferably selected so that the combined use of each of the parts can produce an effect greater than that achieved by using any one component alone in treating the disease or condition. Each component can be in a separate formulation, which can be the same or different.

The term "combination therapy" refers to the administration of two or more therapeutic agents or treatment modes (such as radiotherapy or surgery) to treat the diseases described herein. Such administration includes the co-administration of these therapeutic agents in a substantially simultaneous manner, such as in a single capsule with a fixed ratio of active ingredients. Alternatively, such administration includes the co-administration of each active ingredient in multiple or separate containers (such as tablets, capsules, powders and liquids). The powder and/or liquid can be reconstituted or diluted to the desired dose before administration. In addition, such administration also includes the administration of each type of therapeutic agent in a sequential manner at approximately the same time or at different times. In either case, the treatment regimen will provide the beneficial effects of the pharmaceutical combination in treating the disorders or conditions described herein.

As used herein, "treat" means to slow, interrupt, arrest, alleviate, stop, reduce, or reverse the onset of symptoms, complications, or biochemical signs of a disease; relieve symptoms, or prevent or inhibit further development of a disease, condition, or disorder.

As used herein, "prevent" includes inhibition of the occurrence or development of a disease or disorder or symptoms of a particular disease or disorder.

### II Antibodies

In some embodiments, the anti-OX40L antibodies or antigen-binding fragments of the invention can effectively block the interaction between OX40L and its receptor OX40, such as binding, at both protein and cellular levels, and/or block the activation of OX40 signaling.

In some embodiments, the anti-OX40L antibodies or antigen-binding fragments of the invention can effectively inhibit the activation, proliferation, and/or differentiation of T cells, for example, inhibit the secretion levels of T cells for inflammatory factors such as interleukins like IL-5 and/or IL-3, or inhibit Th2 inflammatory responses.

In some embodiments, the anti-OX40L antibodies or antigen-binding fragments thereof of the invention can maintain the body weight of individuals with graft-versus-host disease (or sustain their weight gain), keep them symptom-free, reduce T cell activation, and/or inhibit the production of their inflammatory cytokines (such as interferons like IFNγ or interleukins like IL-6).

In some embodiments, the anti-OX40L antibody or antigen-binding fragment thereof of the present invention comprises three complementary determining regions (HCDRs) from the heavy chain variable region, HCDR1, HCDR2, and HCDR3.

In some embodiments, the anti-OX40L antibody or antigen-binding fragment thereof of the present invention comprises three complementary determining regions (LCDRs) from the light chain variable region, LCDR1, LCDR2, and LCDR3.

In some embodiments, the anti-OX40L antibody or antigen-binding fragment thereof of the present invention comprises three complementary determining regions (HCDRs) from the heavy chain variable region and three complementary determining regions (LCDRs) from the light chain variable region.

In some aspects, the anti-OX40L antibody or antigen-binding fragment thereof of the present invention comprises a heavy chain variable region (VH). In some aspects, the anti-OX40L antibody or antigen-binding fragment thereof of the present invention comprises a light chain variable region (VL). In some aspects, the anti-OX40L antibody or antigen-binding fragment thereof of the present invention comprises a heavy chain variable region(VH) and a light chain variable region (VL). In some embodiments, the heavy chain variable region comprises three complementary determining regions (CDRs) from the heavy chain variable region, HCDR1, HCDR2, and HCDR3. In some embodiments, the light chain variable region comprises three complementary determining regions (CDRs) from the light chain variable region, LCDR1, LCDR2 and LCDR3.

In some embodiments, the anti-OX40L antibody or its antigen-binding fragment of the present invention further comprises an antibody heavy chain constant region. In some embodiments, the anti-OX40L antibody or its antigen-binding fragment of the present invention further comprises an antibody light chain constant region. In some embodiments, the anti-OX40L antibody or its antigen-binding fragment of the present invention further comprises a heavy chain constant region and a light chain constant region.

In some embodiments, the heavy chain variable region described in the present invention:
(i) comprises or consists of an amino acid sequence having at least 90%, 91%, 92%, 93%, 94%, 95%, 96%, 97%, 98% or 99% identity with an amino acid sequence selected from any one of SEQ ID NO:4 and SEQ ID NO: 14; or
(ii) comprises or consists of an amino acid sequence selected from any one of SEQ ID NO:4 and SEQ ID NO:14; or
(iii) comprises or consists of an amino acid sequence having one or more (preferably no more than 10, more preferably no more than 5, 4, 3, 2, 1) amino acid changes (preferably amino acid substitutions, more preferably amino acid conservative substitutions) compared to an amino acid sequence selected from any one of SEQ ID NO:4 and SEQ ID NO:14, and preferably, the amino acid changes do not occur in the CDR region.

In some embodiments, the light chain variable region described in the present invention
(i) comprises or consists of an amino acid sequence having at least 90%, 91%, 92%, 93%, 94%, 95%, 96%, 97%, 98% or 99% identity with an amino acid sequence selected from SEQ ID NO:8 and SEQ ID NO:18; or
(ii) comprises or consists of an amino acid sequence selected from SEQ ID NO:8 and SEQ ID NO:18; or
(iii) comprises or consists of an amino acid sequence having one or more (preferably no more than 10, more preferably no more than 5, 4, 3, 2, 1) amino acid changes (preferably amino acid substitutions, more preferably amino acid conservative substitutions) compared to an amino acid sequence selected from SEQ ID NO:8 and SEQ ID NO:18, and preferably, the amino acid changes do not occur in the CDR region.

In some embodiments, the three complementary determining regions (HCDRs) from the heavy chain variable region described in the present invention, HCDR1, HCDR2 and HCDR3 are selected from
(i) three complementary determining regions HCDR1, HCDR2 and HCDR3 contained in the VH set forth in SEQ ID NO:4 or 14;
(ii) relative to the sequence of any one of (i), a sequence comprising at least one and no more than 5, 4, 3, 2 or 1 amino acid changes (preferably amino acid substitutions, preferably conservative substitutions) in total in the three HCDR regions,

for example, wherein the HCDRs can be determined according to any scheme for determining CDRs, such as according to Kabat, AbM, Chothia, Contact or IMGT schemes or the combination thereof respectively;
for example, the HCDR1 is determined according to AbM scheme, HCDR2 and HCDR3 are determined according to the Kabat scheme respectively.

In some embodiments, the three complementary determining regions (LCDR) from the light chain variable region described in the present invention, LCDR1, LCDR2 and LCDR3 are selected from
(i) three complementary determining regions LCDR1, LCDR2 and LCDR3 contained in VL set forth in SEQ ID NO:8 or 18, or
(ii) relative to the sequence of any one of (i), a sequence comprising at least one and no more than 5, 4, 3, 2 or 1 amino acid changes (preferably amino acid substitutions, preferably conservative substitutions) in total in the three LCDR regions,

for example, wherein the LCDRs can be determined according to any scheme for determining CDRs, such as according to Kabat, AbM, Chothia, Contact or IMGT schemes or the combination thereof respectively;
for example, the LCDR1, LCDR2 and LCDR3 are determined according to the Kabat scheme respectively.

In some embodiments, the HCDR1 comprises the amino acid sequence set forth in SEQ ID NO: 1 or 11, or consists of the amino acid sequence, or the HCDR1 comprises an amino acid sequence having one, two or three changes (preferably amino acid substitutions, preferably conservative substitutions) compared to the amino acid sequence set forth in SEQ ID NO: 1 or 11.

In some embodiments, the HCDR2 comprises the amino acid sequence set forth in SEQ ID NO:2 or 12, or consists of the amino acid sequence, or the HCDR2 comprises an amino acid sequence having one, two or three changes (preferably amino acid substitutions, preferably conservative substitutions) compared to the amino acid sequence set forth in SEQ ID NO:2 or 12.

In some embodiments, the HCDR3 comprises the amino acid sequence set forth in SEQ ID NO: 3 or 13, or consists of the amino acid sequence, or the HCDR3 comprises an amino acid sequence having one, two or three changes (preferably amino acid substitutions, preferably conservative substitutions) compared to the amino acid sequence set forth in SEQ ID NO: 3 or 13.

In some embodiments, the LCDR1 comprises the amino acid sequence of SEQ ID NO: 5 or 15, or consists of the amino acid sequence, or the LCDR1 comprises an amino acid sequence having one, two or three changes (preferably amino acid substitutions, preferably conservative substitutions) compared to the amino acid sequence of SEQ ID NO: 5 or 15.

In some embodiments, the LCDR2 comprises the amino acid sequence of SEQ ID NO: 6 or 16, or consists of the amino acid sequence, or the LCDR2 comprises an amino acid sequence having one, two or three changes (preferably amino acid substitutions, preferably conservative substitutions) compared to the amino acid sequence of SEQ ID NO: 6 or 16.

In some embodiments, the LCDR3 comprises an amino acid sequence set forth in SEQ ID NO:7 or 17, or consists of the amino acid sequence, or the LCDR3 comprises an amino acid sequence having one, two or three changes (preferably amino acid substitutions, preferably conservative substitutions) compared to the amino acid sequence set forth in SEQ ID NO:7 or 17.

In some embodiments, the anti-OX40L antibodies or antigen-binding fragments of the invention further comprise an antibody heavy chain. In some embodiments, the anti-OX40L antibodies or antigen-binding fragments of the invention further comprise an antibody light chain. In some embodiments, the anti-OX40L antibodies or antigen-binding fragments of the invention further comprise both a heavy and a light chain. In some embodiments, the heavy chain of the antiboty of the present invention comprises the heavy chain variable region and a heavy chain constant region, or consists of the heavy chain variable region and a heavy chain constant region. In some embodiments, the light chain of the antibody of the present invention comprises the light chain variable region and a light chain constant region, or consists of the light chain variable region and a light chain constant region. In some embodiments, the antibody of the invention comprises two heavy chains and two light chains, or consists of two heavy chains and two light chains.

In some embodiments, the heavy chain constant region of the antiboty of the present invention is the heavy chain constant region of IgG1, IgG2, IgG3, or IgG4, preferably the heavy chain constant region of IgG4. In some embodiments, the light chain constant region of the antiboty of the present invention is a lambda or kappa light chain constant region, preferably a kappa light chain constant region.

In some embodiments, the heavy chain constant region of the antiboty of the present invention comprises an Fc region or a mutated Fc region.

In some embodiments, the Fc region is from a human IgG Fc, for example, from human IgG1 Fc, from human IgG2 Fc, from human IgG3 Fc, or from human IgG4 Fc. In one embodiment, the Fc region is from human IgG4. In one embodiment, the Fc region comprises or consists of an amino acid sequence as set forth in SEQ ID NO:28 or an amino acid sequence having at least 90% identity thereto, for example, 95%, 96%, 97%, 99% or higher identity.

In an embodiment, the Fc region is modified in terms of the effector function (e.g., the complement activation function of the Fc region) of the Fc region. In one embodiment, the effector function has been reduced or eliminated relative to the wild-type isotype Fc region. In one embodiment, the effector function is reduced or eliminated by a method selected from the group consisting of using an Fc isotype that naturally has reduced or eliminated effector function and modifying the Fc region. The Fc region may also comprise modifications that alter the binding affinity to one or more Fc receptors.

In one embodiment, the Fc receptor is an Fcy receptor, particularly a human Fcy receptor. In some embodiments, the Fc region comprises mutations that reduce binding to Fcy receptors. The Fc region also comprises mutations that improve antibody stability, for example, mutations that eliminate the heterogeneity of IgG. In some embodiments, the mutations are capable of reducing the likelihood of chain exchange occurring in the antibody in vivo. In some embodiments, the mutation is a substitution of serine at position 228 with proline. Thus, in a preferred embodiment, the Fc region of the invention has an S228P (EU numbering) mutation. In some embodiments, the Fc region of the antiboty or fragment thereof of the present invention is an IgG4 Fc region comprising an S228P mutation.

Thus, in a preferred embodiment, the Fc region of the invention comprises an amino acid sequence as set forth in SEQ ID NO:27, or an amino acid sequence having at least 85%, 90%, 95%, 96%, 97%, 98%, 99% identity to the amino acid sequence shown in SEQ ID NO:27 and having an S228P mutation.

In some preferred embodiments, the heavy chain constant region of the antiboty of the present invention
(i) comprises or consists of an amino acid sequence having at least 85%, 90%, 91%, 92%, 93%, 94%, 95%, 96%, 97%, 98% or 99% identity to an amino acid sequence selected from SEQ ID NO:21 or 29;
(ii) comprises or consists of the amino acid sequence selected from SEQ ID NO:21 or 29; or
(iii) comprises or consists of an amino acid sequence having one or more (preferably no more than 20 or 10, more preferably no more than 5, 4, 3, 2, 1) amino acid changes (preferably amino acid substitutions, more preferably conservative amino acid substitutions) compared to an amino acid sequence selected from SEQ ID NO:21 or 29.

In some preferred embodiments, the heavy chain constant region of the antiboty of the present invention comprises an amino acid sequence having at least 85%, 90%, 91%, 92%, 93%, 94%, 95%, 96%, 97%, 98% or 99% identity to the amino acid sequence of SEQ ID NO:21 and having an S228P mutation. In some preferred embodiments, the heavy chain constant region of the antiboty of the present invention comprises or consists of the amino acid sequence of SEQ ID NO:21.

In some embodiments, the light chain constant region of the antiboty of the present invention
(i) comprises or consists of an amino acid sequence having at least 85%, 90%, 91%, 92%, 93%, 94%, 95%, 96%, 97%, 98% or 99% identity to the amino acid sequence of SEQ ID NO:22;
(ii) comprises or consists of the amino acid sequence of SEQ ID NO:22; or
(iii) comprises or consists of an amino acid sequence having one or more (preferably no more than 20 or 10, more preferably no more than 5, 4, 3, 2, 1) amino acid changes (preferably amino acid substitutions, more preferably conservative amino acid substitutions) compared to the amino acid sequence of SEQ ID NO:22.

In some specific embodiments, the anti-OX40L antibody of the present invention comprises a first heavy chain complementary determining region (HCDR1), a second heavy chain complementary determining region (HCDR2), a third heavy chain complementary determining region (HCDR3), and a first light chain complementary determining region (LCDR1), a second light chain complementary determining region (LCDR2), and a third light chain complementary determining region (LCDR3), wherein
(i) the HCDR1, HCDR2, HCDR3, and LCDR1, LCDR2, and LCDR3 comprise the amino acid sequences set forth in SEQ ID NO:1, SEQ ID NO:2, SEQ ID NO:3, SEQ ID NO:5, SEQ ID NO:6, and SEQ ID NO:7 respectively, or consist of the amino acid sequences set forth in SEQ ID NO:1, SEQ ID NO:2, SEQ ID NO:3, SEQ ID NO:5, SEQ ID NO:6, and SEQ ID NO:7 respectively; or
(ii) the HCDR1, HCDR2, HCDR3, and LCDR1, LCDR2, and LCDR3 comprise the amino acid sequences set forth in SEQ ID NO:11, SEQ ID NO:12, SEQ ID NO:13, SEQ ID NO:15, SEQ ID NO:16, and SEQ ID NO:17 respectively, or consist of the amino acid sequences set forth in SEQ ID NO:11, SEQ ID NO:12, SEQ ID NO:13, SEQ ID NO:15, SEQ ID NO:16, and SEQ ID NO:17 respectively.

In some embodiments, the VH of the present invention comprises HCDR1, HCDR2, HCDR3, and the VL comprises LCDR1, LCDR2, and LCDR3, the HCDR1, HCDR2, HCDR3 comprise the amino acid sequences set forth in SEQ ID NO:1, SEQ ID NO:2, SEQ ID NO:3 respectively, or consist of the amino acid sequences set forth in SEQ ID NO:1, SEQ ID NO:2, SEQ ID NO:3, the LCDR1, LCDR2 and LCDR3 comprises the amino acid sequence set forth in SEQ ID NO:5, SEQ ID NO:6, and SEQ ID NO:7 respectively, or consist of the amino acid sequences set forth in SEQ ID NO:5, SEQ ID NO:6, SEQ ID NO:7 respectively.

In some embodiments, the VH of the present invention comprises HCDR1, HCDR2, HCDR3, and the VL comprises LCDR1, LCDR2, and LCDR3, wherein the HCDR1, HCDR2, HCDR3 comprise the amino acid sequences set forth in SEQ ID NO:11, SEQ ID NO:12, SEQ ID NO:13 respectively, or consist of the amino acid sequences set forth in SEQ ID NO:11, SEQ ID NO:12, SEQ ID NO:13, the LCDR1, LCDR2 and LCDR3 comprises or consists of the amino acid sequence set forth in SEQ ID NO:15, SEQ ID NO:16, and SEQ ID NO:17 respectively, or consist of the amino acid sequences set forth in SEQ ID NO:15, SEQ ID NO: 16, SEQ ID NO: 17 respectively.

In some embodiments, the antibody or antigen-binding fragment thereof of the present invention comprises
(i) a VH comprising or consisting of the amino acid sequence set forth in SEQ ID NO: 4, or an amino acid sequence having at least 90%, 91%, 92%, 93%, 94%, 95%, 96%, 97%, 98% or 99% identity thereto; and/or a VL comprising or consisting of the amino acid sequence set forth in SEQ ID NO: 8, or an amino acid sequence having at least 90%, 91%, 92%, 93%, 94%, 95%, 96%, 97%, 98% or 99% identity thereto; or
(ii) a VH comprising or consisting of the amino acid sequence set forth in SEQ ID NO: 14, or an amino acid sequence having at least 90%, 91%, 92%, 93%, 94%, 95%, 96%, 97%, 98% or 99% identity thereto; and/or a VL comprising or consisting of the amino acid sequence set forth in SEQ ID NO: 18, or an amino acid sequence having at least 90%, 91%, 92%, 93%, 94%, 95%, 96%, 97%, 98% or 99% identity thereto.

In some embodiments, the antibody or antigen-binding fragment thereof of the present invention comprises a heavy chain variable region and a light chain variable region, wherein
(i) the heavy chain variable region comprises or consists of the amino acid sequence set forth in SEQ ID NO: 4, and the light chain variable region comprises or consists of the amino acid sequence set forth in SEQ ID NO: 8; or
(ii) the heavy chain variable region comprises or consists of the amino acid sequence set forth in SEQ ID NO: 14, and the light chain variable region comprises or consists of the amino acid sequence set forth in SEQ ID NO: 18.

In some embodiments, the anti-OX40L antibody or antigen-binding fragment thereof of the present invention further comprises the antibody heavy chain. In some embodiments, the anti-OX40L antibody or antigen-binding fragment thereof of the present invention further comprises the antibody light chain. In some embodiments, the anti-OX40L antibody or antigen-binding fragment thereof of the present invention further comprises the heavy chain and the light chain.

In some embodiments, the heavy chain
(i) comprises an amino acid sequence having at least 85%, 90%, 91%, 92%, 93%, 94%, 95%, 96%, 97%, 98% or 99% identity with an amino acid sequence selected from SEQ ID NO: 9 or 19 or consists of the amino acid sequence; or
(ii) comprises an amino acid sequence selected from SEQ ID NO: 9 or 19 or consists of the amino acid sequence; or
(iii) comprises an amino acid sequence having one or more (preferably no more than 20 or 10, more preferably no more than 5, 4, 3, 2, 1) amino acid changes (preferably amino acid substitutions, more preferably conservative amino acid substitutions) compared to an amino acid sequence selected from SEQ ID NO: 9 or 19 or consists of the amino acid sequence.

In some embodiments, the light chain
(i) comprises an amino acid sequence having at least 85%, 90%, 91%, 92%, 93%, 94%, 95%, 96%, 97%, 98% or 99% identity with an amino acid sequence selected from SEQ ID NO: 10 or 20 or consists of the amino acid sequence; or
(ii) comprises an amino acid sequence selected from SEQ ID NO: 10 or 20 or consists of the amino acid sequence; or
(iii) comprises an amino acid sequence having one or more (preferably no more than 20 or 10, more preferably no more than 5, 4, 3, 2, 1) amino acid changes (preferably amino acid substitutions, more preferably conservative amino acid substitutions) compared to an amino acid sequence selected from SEQ ID NO: 10 or 20 or consists of the amino acid sequence.

In some embodiments, the antibody or antigen-binding fragment thereof of the present invention comprises a heavy chain and a light chain, wherein
(i) the heavy chain comprises or consists of an amino acid sequence having at least 85%, 90%, 91%, 92%, 93%, 94%, 95%, 96%, 97%, 98% or 99% identity with the amino acid sequence set forth in SEQ ID NO:9, and/or the light chain comprises or consists of an amino acid sequence having at least 85%, 90%, 91%, 92%, 93%, 94%, 95%, 96%, 97%, 98% or 99% identity with the amino acid sequence set forth in SEQ ID NO:10; or
(ii) the heavy chain comprises or consists of an amino acid sequence having at least 85%, 90%, 91%, 92%, 93%, 94%, 95%, 96%, 97%, 98% or 99% identity with the amino acid sequence set forth in SEQ ID NO:19, and/or the light chain comprises or consists of an amino acid sequence having at least 85%, 90%, 91%, 92%, 93%, 94%, 95%, 96%, 97%, 98% or 99% identity with the amino acid sequence set forth in SEQ ID NO:20.

In some embodiments, the antibody or antigen-binding fragment thereof of the present invention comprises a heavy chain and a light chain, wherein
(i) the heavy chain comprises or consists of the amino acid sequence of SEQ ID NO:9, and the light chain comprises or consists of the amino acid sequence of SEQ ID NO: 10; or
(ii) the heavy chain comprises or consists of the amino acid sequence of SEQ ID NO:19, and the light chain comprises or consists of the amino acid sequence of SEQ ID NO:20.

In an embodiment of the present invention, the amino acid changes described herein include amino acid substitutions, insertions or deletions. Preferably, the amino acid changes described herein are amino acid substitutions, preferably conservative substitutions.

In a preferred embodiment, the amino acid changes described herein occur in a region outside the CDR (e.g., in the FR). More preferably, the amino acid changes described herein occur in a region outside the heavy chain variable region and/or outside the light chain variable region.

In certain embodiments, the antibodies provided herein are altered to increase or decrease the degree to which the antibodies are glycosylated. The addition or deletion of glycosylation sites to an antibody can be conveniently achieved by altering the amino acid sequence to create or remove one or more glycosylation sites. When the antibody comprises an Fc region, the carbohydrates attached thereto can be altered. In some applications, modifications to remove unwanted glycosylation sites can be useful, such as removing fucose motifs to improve antibody-dependent cellular cytotoxicity (ADCC) function. In other applications, galactosylation modifications can be performed to modify complement-dependent cytotoxicity (CDC).

In certain embodiments, it may be desirable to produce cysteine-engineered antibodies, such as "thioMAbs," in which one or more residues of an antibody are replaced with cysteine residues.

In certain embodiments, the antibodies provided herein may be further modified to contain other non-protein moieties known in the art and readily available. Moieties suitable for antibody derivatization include, but are not limited to, water-soluble polymers. Non-limiting examples of water-soluble polymers include, but are not limited to, polyethylene glycol (PEG), ethylene glycol/propylene glycol copolymers, carboxymethyl cellulose, dextran, polyvinyl alcohol, polyvinyl pyrrolidone, poly-1,3-dioxane, poly-1,3,6-trioxane, ethylene/maleic anhydride copolymers, polyamino acids (homopolymers or random copolymers), and dextran or poly (n-vinyl pyrrolidone) polyethylene glycol, propylene glycol homopolymers, polypropylene oxide/ethylene oxide copolymers, polyoxyethylated polyols (e.g., glycerol), polyvinyl alcohol, and mixtures thereof.

In some embodiments, the anti-OX40L antibodies or antigen-binding fragments thereof of the present invention have one or more of the following properties:
(i) exhibiting the same or similar binding affinity and/or specificity to OX40L as the antibodies of the present invention;
(ii) inhibiting (e.g., competitively inhibiting) the binding to OX40L of the antibodies of the present invention;
(iii) binding to the same or overlapping epitopes as the antibodies of the present invention;
(iv) competing for binding to OX40L with the antibodies of the present invention;
(v) having one or more biological properties of the antibodies of the present invention.

In some embodiments, the anti-OX40L antibody of the present invention is an antibody in the form of IgG1, an antibody in the form of IgG2, an antibody in the form of IgG3, or an antibody in the form of IgG4, preferably, an antibody in the form of IgG4.

In some embodiments, the anti-OX40L antibody is a monoclonal antibody.

In some embodiments, the anti-OX40L antibody is humanized.

In some embodiments, the anti-OX40L antibod is a chimeric antibody.

In some embodiment, the anti-OX40L antibody of the present invention also encompasses antibody fragments thereof (e.g., antigen-binding fragments), preferably selected from the following antibody fragments: Fab, Fab', Fab'-SH, Fv, single-chain antibodies (e.g., scFv), (Fab')2, single-domain antibodies such as VHH, dAb (domain antibody), bivalent antibody, or linear antibodies.

In some embodiments, the anti-OX40L antibody of the present invention also encompasses multispecific antibodies such as bispecific antibodies that specifically bind to OX40L.

In some embodiments, the anti-OX40L antibody of the present invention is a full-length antibody.

### III. Nucleic acids of the present invention and host cells containing the same

In an aspect, the present invention provides a nucleic acid encoding any chain or any monomer or domain of the antibody or antigen-binding fragment thereof of the present invention. A polynucleotide sequence encoding each chain can be produced by methods well known in the art. For example, when expressed from a suitable expression vector, the polypeptide encoded by the nucleic acid can show binding ability to human OX40L antigen. For example, in some embodiments, the nucleic acid encoding the variable region of the heavy chain and/or light chain is operably linked in frame with the nucleic acid encoding the constant region of the heavy chain and/or light chain, thereby generating nucleic acids encoding the heavy chain and/or of the antibody when expressed from a suitable expression vector.

In an aspect, the present invention provides nucleic acids encoding any anti-OX40L antibody or fragment thereof described herein. The nucleic acid may comprise a nucleic acid encoding the amino acid sequence of the light chain variable region and/or heavy chain variable region of the antibody, or comprise a nucleic acid encoding the amino acid sequence of the light chain and/or heavy chain of the antibody.

For example, the nucleic acid of the present invention comprises a nucleic acid encoding an amino acid sequence selected from any one of SEQ ID NOs: 4, 8-10, 14 and 18-20, or a nucleic acid encoding an amino acid sequence having at least 85%, 90%, 91%, 92%, 93%, 94%, 95%, 96%, 97%, 98% or 99% identity with an amino acid sequence selected from any one of SEQ ID NOs: 4, 8-10, 14 and 18-20. As is apparent to those skilled in the art, each antibody or polypeptide amino acid sequence can be encoded by a variety of nucleic acid sequences due to codon degeneracy. The nucleic acid sequence encoding the molecule of the present invention can be produced by methods well known in the art, such as by de novo solid phase DNA synthesis, or by PCR amplification. For ease of production and purification, a secretory signal peptide, and/or a tag peptide that facilitates purification can be fused to the N-terminus of the heavy chain and/or light chain of the antibody.

The present invention also provides a vector comprising the nucleic acid of the present invention. In one embodiment, the vector is an expression vector, such as a eukaryotic expression vector. Vectors include, but are not limited to, viruses, plasmids, cosmids, λ phages or yeast artificial chromosomes (YACs). In a preferred embodiment, the expression vector of the present invention is a pcDNA vector, such as a pcDNA3.1 expression vector.

In one embodiment, provided herein is a host cell comprising the vector. The present invention also provides a host cell comprising the nucleic acid or the vector. Host cells suitable for replication and support expression of the antibodies of the present invention are well known in the art. Such cells can be transfected or transduced with a specific expression vector, and a large number of vector-containing cells can be grown for inoculation of large-scale fermenters, thereby obtaining sufficient amounts of antibodies for clinical applications. Suitable host cells for cloning or expressing vectors encoding antibodies include prokaryotic or eukaryotic cells described herein. For example, antibodies can be produced in bacteria, especially when glycosylation and Fc effector functions are not required. After expression, the antibodies can be separated from the bacterial cell paste in the soluble fraction and can be further purified.

In one embodiment, the host cell is eukaryotic. In another embodiment, the host cell is selected from a yeast cell, a mammalian cell (e.g., CHO cell (e.g., CHO-S or CHO-K) or 293 cell (e.g., 293F or HEK293 cell)) or other cells suitable for preparing antibodies or fragments thereof. In one embodiment, the host cell is prokaryotic, for example, a bacterium, such as E. coli.

For example, eukaryotic microorganisms such as filamentous fungi or yeast are suitable cloning or expression hosts for vectors encoding antibodies. For example, fungal and yeast strains whose glycosylation pathways have been "humanized" result in the production of antibodies with partially or fully human glycosylation patterns. Host cells suitable for expressing glycosylated antibodies are also derived from multicellular organisms (invertebrates and vertebrates). Vertebrate cells can also be used as hosts. For example, mammalian cell lines modified to be suitable for suspension growth can be used. Other examples of useful mammalian host cell lines are monkey kidney CV1 lines transformed with SV40(COS-7); human embryonic kidney lines (HEK293, 293F or 293T cells), etc. Other useful mammalian host cell lines include Chinese hamster ovary (CHO) cells, including DHFR⁻CHO cells, CHO-S cells, ExpiCHO, etc.; and myeloma cell lines such as Y0, NS0 and Sp2/0. Mammalian host cell lines suitable for producing antibodies are known in the art.

### IV. Production and purification of antibody molecules of the present invention

In one embodiment, the present invention provides a method for preparing an antibody molecule or fragment thereof (preferably an antigen-binding fragment) of the present invention, wherein the method comprises culturing the host cell under conditions suitable for expressing a nucleic acid encoding an antibody molecule or fragment thereof (preferably an antigen-binding fragment) of the present invention, and optionally isolating the antibody or fragment thereof (e.g., an antigen-binding fragment). In a certain embodiment, the method further comprises recovering the antibody molecule or fragment thereof (e.g., an antigen-binding fragment) of the present invention from the host cell.

The polynucleotide encoding the polypeptide chain of the antibody of the present invention may be inserted into one or more vectors for further cloning and/or expression in a host cell. Expression vectors may be constructed using methods well known to those skilled in the art. Once an expression vector comprising one or more nucleic acid molecules of the present invention has been prepared for expression, the expression vector may be transfected or introduced into a suitable host cell. A variety of techniques may be used to achieve this purpose, for example, protoplast fusion, calcium phosphate precipitation, electroporation, retroviral transduction, viral transfection, gene gun, liposome-based transfection or other conventional techniques.

The antibody molecules prepared as described herein can be purified by known prior art techniques such as high performance liquid chromatography, ion exchange chromatography, gel electrophoresis, affinity chromatography, size exclusion chromatography, etc. The actual conditions used to purify a particular protein will also depend on factors such as net charge, hydrophobicity, hydrophilicity, and these will be apparent to those skilled in the art. The purity of the antibody molecules of the invention can be determined by any of a variety of well-known analytical methods, including size exclusion chromatography, gel electrophoresis, high performance liquid chromatography, and the like.

### V. Assays

The anti-OX40L antibodies provided herein can be identified, screened, or characterized for their physical/chemical properties and/or biological activity by a variety of assays known in the art.

The invention also provides assays for identifying anti-OX40L antibodies with biological activity. Biological activity may include, for example, binding to OX40L (e.g., binding to human OX40L), inhibition of the OX40/OX40L signaling pathway, blocking the binding of OX40 to OX40L, inhibition of T cell activation, proliferation, and/or differentiation (e.g., differentiation to Th2), inhibition of inflammatory cytokine production, and therapeutic effects on immune system diseases (e.g., graft-versus-host disease). Antibodies with such biological activity in vivo and/or in vitro are also provided.

For the assays of the above biological activities, reference can be made to the exemplary assay methods given in the examples.

It can be understood that the immunoconjugates of the invention can be used to replace or supplement the anti-OX40L antibodies for any of the above assays.

It can be understood that a combination of anti-OX40L antibodies and other therapeutic agents can be used for any of the above assays.

### VI. IMMUNOTOXINS

In some embodiments, the invention provides immunoconjugates, which comprises any anti-OX40L antibody provided herein and another agent, such as an active agent or label suitable for forming an immunoconjugate with the OX40L antibody. In some embodiments, the active agent suitable for forming an immunoconjugate with the OX40L antibody may be, for example, a toxin, small molecule drug, cytotoxic agent, cellular apoptotic agent, chelating agent, immune modulator, such as an anti-inflammatory agent or immunosuppressive agent.

In some embodiments, the immunoconjugate is an antibody-drug conjugate (ADC).

### VII. Pharmaceutical compositions and pharmaceutical formulations

In some embodiments, the present invention provides a composition comprising any anti-OX40L antibody or fragment thereof (preferably an antigen-binding fragment thereof) or the immunoconjuage thereof described herein, preferably the composition is a pharmaceutical composition. In one embodiment, the composition further comprises a pharmaceutically acceptable excipient. In one embodiment, the composition, e.g., a pharmaceutical composition, comprises an anti-OX40L antibody or fragment thereof or the immunoconjugate thereof of the present invention in combination with one or more other therapeutic agents.

The present invention also includes compositions (including pharmaceutical compositions or pharmaceutical formuations) comprising anti-OX40L antibodies or or the immunoconjugate thereof, or compositions (including pharmaceutical compositions or pharmaceutical formuations) comprising polynucleotides encoding anti-OX40L antibodies or fragments thereof. In certain embodiments, the composition comprises one or more antibodies or fragments thereof that bind to OX40L, or one or more polynucleotides encoding one or more anti-OX40L antibodies or fragments thereof. These compositions may also comprise suitable pharmaceutically acceptable excipients, such as pharmaceutical carriers, pharmaceutical vehicles, including buffers, as known in the art.

As used herein, "pharmaceutically acceptable carriers" include any and all solvents, dispersion media, isotonic agents, absorption delaying agents, and the like that are physiologically compatible. For the use of pharmaceutically acceptable excipients and their uses, see also "Handbook of Pharmaceutical Excipients", 8th edition, R.C. Rowe, P.J. Seskey and S.C. Owen, Pharmaceutical Press, London, Chicago.

The compositions of the present invention may be in a variety of forms. These forms for example include liquid, semisolid and solid dosage forms, such as liquid solutions (e.g., injectable solutions and infusible solutions), powders or suspensions, liposomes and suppositories. The preferred form depends on the intended mode of administration and therapeutic use.

Pharmaceutical formulations containing the antibodies described herein may be prepared by mixing the antibodies of the present invention having the desired purity with one or more optional pharmaceutically acceptable excipients, preferably in the form of a lyophilized formulation or a water solution.

The pharmaceutical compositions or formulations of the present invention may also contain more than one active ingredient, which is required for the specific indication being treated, preferably those with complementary activities that do not adversely affect each other. For example, it is desirable to also provide other therapeutic agents, such as cytokines, small molecule drugs, immune modulators (e.g., immunosuppressive agents or anti-inflammatory agents), or other antibodies, etc. The active ingredients are present in suitable combinations in amounts effective for the intended purpose.

Sustained release formulations can be prepared. Suitable examples of sustained release formulations include a semipermeable matrix of a solid hydrophobic polymer containing an antibody, the matrix being in the form of a shaped article, such as a film or microcapsule.

### VIII. Pharmaceutical Combinations and Kits

In some embodiments, the present invention also provides a pharmaceutical combination or a pharmaceutical combination product comprising the anti-OX40L antibody or a fragment thereof (preferably an antigen-binding fragment) or the immunoconjugate thereof of the present invention, and one or more other therapeutic agents(such as cytokines, small molecule drugs, immune modulators (e.g., immunosuppressive agents or anti-inflammatory agents), or other antibodies, etc).

Another object of the present invention is to provide a kit comprising the pharmaceutical combination of the present invention, preferably the kit is in the form of a pharmaceutical dosage unit. Thus, the dosage unit can be provided according to the dosing regimen or the interval of drug administration.

In one embodiment, the kit of the present invention comprises in the same package:
- A first container containing a pharmaceutical composition comprising an anti-OX40L antibody or a fragment thereof;
- A second container containing a pharmaceutical composition comprising other therapeutic agents.

### IX. Uses and Methods

In some embodiments, the antibodies of the invention are used as monotherapy or in combination therapy to prevent or treat diseases or conditions in an individual that are associated with abnormal activation of OX40L/OX40-mediated pathways.

In some embodiments, the antibodies of the invention are capable of inhibiting T cell activation responses and thereby reducing related immune responses, and reducing diseases or symptoms associated with hyperreactivity of an autoimmune by blocking the OX40L-OX40 signaling.

In some embodiments, patients with the disease or condition have hyperreactivity of an immune system mediated by OX40L/OX40 compared to healthy individuals. In some embodiments, the cells (e.g., antigen-presenting cells) of patients with the disease or condition have increased expression of OX40L and/or the cells (e.g., T cells, such as activated T cells) of patients have increased expression of OX40, for example, compared to the corresponding cells of healthy individuals.

In some embodiments, the disease or condition is selected from autoimmune diseases or graft-versus-host disease. In some embodiments, autoimmune diseases include, but are not limited to, allergic dermatitis, asthma, systemic lupus erythematosus, Sjogren's syndrome, immune thrombocytopenic purpura, multiple sclerosis, lupus nephritis, amyotrophic lateral sclerosis, rheumatoid arthritis, non-rheumatoid arthritis, contact dermatitis, hyper IgE syndrome, inflammatory bowel disease, myasthenia gravis, Graves' disease, hemolytic anemia, psoriasis, atopic dermatitis (e.g., moderate to severe atopic dermatitis), allergic asthma, or idiopathic inflammatory disease (CN106459196B).

In some embodiments, the antibodies, or antibody fragments, or immunoconjugates, or compositions, or products of the invention delay the onset of the condition and/or symptoms associated with the condition.

In some embodiments, the present invention provides a use of the anti-OX40L antibody or fragment thereof of the present invention or an immunoconjugate or composition comprising the same in the production or preparation of a medicine, which is used for the uses described herein, such as for preventing or treating the relevant diseases or conditions mentioned herein.

In some embodiments, the prevention or treatment methods described herein also include administering to the subject or individual the antibody molecule or pharmaceutical composition or immunoconjugate disclosed herein in combination with one or more other therapies, such as treatment modalities and/or other therapeutic agents. In some embodiments, anti-OX40L antibodies or fragments thereof (as well as immunoconjugates, compositions, pharmaceutical compositions, formulations, etc. comprising the same) can also be administered in combination with one or more other therapies, such as treatment modalities and/or other therapeutic agents, for the uses described herein, such as for preventing and/or treating the relevant diseases or conditions mentioned herein.

In some embodiments, the therapeutic agents are selected from the group consisting of cytokines, small molecule drugs, immune modulators (e.g., immunosuppressive agents or anti-inflammatory agents), or other antibodies.

In some embodiments, the antibodies or antigen-binding fragments described herein can be combined with other antibodies or antigen-binding fragments thereof for separate administration, for example, separately as separate antibodies, or for administration when connected (e.g., as bispecific or multispecific antibody molecules).

Such combination therapies encompass administration in combination (e.g., two or more therapeutic agents are contained in the same formulation or in separate formulations), and separate administration, in which case administration of the antibodies of the invention may occur before, simultaneously with, and/or after administration of the other therapeutic agents and/or therapies.

The antibodies or fragments thereof of the invention (and immunoconjugates, compositions, pharmaceutical compositions, formulations, combination products, etc. comprising the same) may be administered by any suitable method, including parenteral administration, and, if desired for local treatment, intralesional administration. Parenteral injection or infusion includes intramuscular, intravenous, intra-arterial, intraperitoneal, or subcutaneous injection or infusion.

### X. Methods and compositions for diagnosis and detection

In one aspect, the present invention also relates to methods for diagnosis and detection of the antibodies or antigen-binding fragments thereof of the present invention and compositions for diagnosis and detection comprising the same.

In certain embodiments, any anti-OX40L antibody or fragment thereof (preferably antigen-binding fragment) provided herein can be used to detect the presence of OX40L, in a biological sample.

The term "detection" as used herein includes quantitative or qualitative detection. Exemplary detection methods may involve immunohistochemistry, immunocytochemistry, flow cytometry (e.g., FACS), magnetic beads complexed with antibody molecules, ELISA assays, PCR-techniques (e.g., RT-PCR). In certain embodiments, the biological sample is blood, serum, or other liquid sample of biological origin. In certain embodiments, the biological sample comprises cells or tissues.

In one embodiment, an anti-OX40L antibody or fragment thereof for use in a diagnostic or detection method is provided.

In another aspect, a method for detecting the presence of OX40L, in a biological sample is provided. In certain embodiments, the method comprises detecting the presence of OX40L protein in a biological sample. In certain embodiments, OX40L is human OX40L. In certain embodiments, the method comprises contacting a biological sample with an anti-OX40L antibody or fragment thereof as described herein under conditions that allow the anti-OX40L antibody or fragment thereof to bind to OX40L, and detecting whether a complex is formed between the anti-OX40L antibody or fragment thereof and OX40L. The formation of a complex indicates the presence of OX40L. The method can be an in vitro or in vivo method. In one embodiment, the anti-OX40L antibody or fragment thereof is used to select a subject suitable for treatment with the anti-OX40L antibody or fragment thereof, for example, wherein OX40L, is a biomarker for selecting the subject.

In some embodiments, a labeled anti-OX40L antibody or fragment thereof is provided. Labels include, but are not limited to, labels or moieties (such as fluorescent labels, chromophore labels, electron-dense labels, chemiluminescent labels, and radioactive labels) that can be directly detected, and moieties that can be indectly detected, such as enzymes or ligands, for example, by enzymatic reactions or molecular interactions.

In some embodiments provided herein, the sample is obtained prior to treatment with an anti-OX40L antibody or fragment thereof. In some embodiments, the sample is obtained prior to other therapies. In some embodiments, the sample is obtained during treatment with other therapies, or after treatment with other therapies.

In some embodiments, OX40L is detected prior to treatment, e.g., prior to initiation of treatment or prior to a certain treatment after a treatment interval.

In some embodiments, a method for treating the diseases of the invention is provided, the method comprising: testing for the presence of OX40L in a subject (e.g., a sample) (e.g., a subject sample) to thereby determine an OX40L value, comparing the OX40L value with a control value, and if the OX40L value is greater than the control value, administering to the subject a therapeutically effective amount of an anti-OX40L antibody or fragment thereof (e.g., the anti-OX40L antibody or fragment described herein), optionally in combination with one or more other therapies, thereby treating the disease.

These and other aspects and embodiments of the present invention are described in the figures(a brief description of the figures enclosed) and the detailed description of the invention and are exemplified in the following examples. Any or all of the features discussed above and throughout this application may be combined in various embodiments of the present invention. The present invention is further illustrated by the following examples. However, it should be understood that the examples are described in an illustrative rather than a limiting manner and that various modifications may be made by those skilled in the art.

### Examples

Equipment: Thermo Fisher MULTISKAN-FC (Examples 1-3)
Equipment: Fluorescence quantitative microplate reader SPARK (Example 1)
Equipment: BD FACSymphony A3 Flow Cytometer (Example 4)

### Reagents and raw materials:

| Name | Manufactuer | Catalog number |
|---|---|---|
| PBS(1X) | Gibco | 10010-023 |
| BSA | Sangon Biotech (Shanghai) Co., Ltd. | A500023-0100 |
| Tween | Sangon Biotech (Shanghai) Co., Ltd. | A600560-0500 |
| TMB developing solution | Solarbio | PR1200 |
| ELISA stop solution | Solarbio | C1058 |
| human OX40 ligand, his tag (OX40L recombinant protein) | Acro | OXL-H52Q8 |
| human OX40,Fc,Avitag (OX40R-Avi) | Acro | OX0-H82F7 |
| Streptavidin-HRP | Biolegend | 405210 |
| RPMI medium 1640 (1x) | Shanghai BasalMedia Technologies Co., Ltd. | L210KJ |
| FBS | Gibco | 10099141C |
| Bio-Glo^{™} Luciferase Assay System | Promega | G7940 |
| RPMI medium 1640 (1x) | Shanghai BasalMedia Technologies Co., Ltd. | L210KJ |
| FBS (Fetal bovine serum) | Gibco | 10099141C |
| Pan T Cell Isolation Kit | Miltenyi Biotec | 130-096-535 |
| Human IL-2 DuoSet ELISA | R&D | DY202 |
| X-VIVO 15 | LONZA | 04-418Q |
| Recombinant hTSLP | Sino Biological | 16135-H08H |
| EasySep^{™} Human Naive CD4+ T Cell Isolation Kit | Stemcell | 19555 |
| EasySep^{™} Human Myeloid DC Enrichment Kit | Stemcell | 19061 |
| Anti-Human CD3 mAb (OKT3), Ultra-low end | Acro Biosystems | CDE-M120A-1MG |
| Anti-human CD28 (Clone CD28.2) | 16-0289-85 | eBioscience^{™} |
| Human IL-5 DuoSet ELISA | R&D | DY205 |
| Human IL-13 ELISA BASIC kit (HRP) | Mabtech | 3471-1H-6-20 |
| EDTA | Thermo | 15575020 |
| LIVE/DEAD Fixable Far Red Dead Cell Stain Kit | Thermo | L10120 |
| PE Mouse Anti-NHP CD45 | BD | 552833 |
| Brilliant Violet 711 anti-human CD8a | Biolegend | 301044 |
| PE-Cy7 Mouse Anti-Human CD4 | BD | 560644 |
| PerCP-Cy5.5 Mouse Anti-Human CD3 | BD | 560835 |
| Brilliant Violet 421 anti-human/mouse Granzyme B | Biolegend | 396414 |

### Example 1: Inhibition of anti-OX40L antibody molecules on binding of OX40 to OX40L

By adopting a hybridoma technology, a cell line of Human GS-CHO cells highly expressing Human OX40L or a Human OX40L fusion protein (Human OX40 Ligand/TNFSF4 Protein, His Tag (active trimer) (MALS verified)), Acro) is used for immunizing a mouse. Spleen of the sacrificed mouse is taken and digested into a single-cell suspension. The spleen cell is hybridized with myeloma cells with proliferation capacity, and the hybridoma cells capable of expressing positive antibodies are obtained under the action of a selective medium. And further screening by in vitro flow cytometry and ELISA methods to obtain the murine antibody capable of binding OX40L and blocking OX40-OX40L interaction.

### CHO-OX40L cell preparation

The nucleic acid sequence encoding Human OX40L (SEQ ID NO:30) was constructed in pXC17.4 plasmid and the plasmid was cleaved with Pvu I (NEB) to obtain a linear vector. And carrying out electrotransfection with GS-CHO cells in good growth state, wherein the system comprises 40 µg of vector and 1×10⁷ cells. The cells were transfered to GS-CHO fresh culture mediun without GlutaMax after the electrotransfection was finished. When GS-CHO growed normally, L-methionine sulfoxide imine was into the culture system, and the cells were screened. After the cell viability recovered to 95% or more, the cells were sorted by flow cytometer to obtain GS-CHO-OX40L (CHO-OX40L).

### Immunization

Balb/c mice (purchased from Beijing Vital River Laboratory Animal Technology Co., Ltd.) were immunized once every two weeks for three times with a cell line **(CHO-OX40L)** of Human GS-CHO cells highly expressing Human OX40L or a Human OX40L fusion protein (Human OX40 Ligand/TNFSF4 Protein, His Tag (active trimer) (MALS verify)), Acro).

### Cell fusion

When the serum titer meets the requirement, the spleen of a mouse is picked to prepare a B lymphocyte suspension, and the B lymphocyte suspension is mixed with SP2/0 myeloma cells (ATCC) in a ratio of 1 : 2 - 1 : 1 to perform electrofusion. After the electrofusion, the cells were transferred from the electrode dish into a 50ml centrifuge tube and diluted to 1 ~ 2×10⁴ cells/ml with HAT-containing medium, and 100µl of cell suspension was added to each well of a 96-well plate. The medium was changed into selection medium at day 7 after the fusion, and after culture for 10 days (or longer, depending on the cell growth state), flow cytometry (FACS) detection was carried out to select positive clones.

### Screening for hybridoma positive clones (FACS)

Hybridoma cells specifically expressing anti-OX40L antibody were screened by flow cytometry (FACS). Cells to be detected (GS-CHO humanOX40L) were counted and diluted to 1×10⁶ cells/ml and 100µl/well of which was added to a U-bottom 96-well plate and centrifuged at 500g for 5min to remove the cell culture medium. The hybridoma culture supernatant in 96-well plate was added to a U-plate and the cells were resuspended in 100µl per well and allowed to stand on ice for 30 min. The supernatant was removed by centrifugation at 500g for 5min, and the cells were washed once with PBS. PBS was removed at 500g for 5min, 100µl of FITC-labeled secondary antibody against mouse Fab (Jackson Immunoresearch, Cat #115-545-006) (1:500 diluted in PBS) was added to each well, and 100µl of PE-labeled secondary antibody against human Fc (Biolegend, Cat #409304) was added for positive control antibody. The mixtures were incubated on ice in the dark for 30min. The supernatant was removed by centrifugation at 500g for 5min and the cells were washed once with PBS. The cells were resuspended in 50µl of 1×PBS and detected by FACS.

### Functional screening of hybridoma cells blocking OX40/OX40L

The above hybridoma positive clones were selected and tested for activity in blocking OX40 binding to OX40L. Based on the blocking results, subclones of the positive clone were selected.

### Positive hybridoma cell subcloning

200µl of culture medium was added to each well of a 96-well plate. The culture medium was prepared by replacing HAT with HT (Gibco, Cat#11067-030) based on the selection medium, and the rest of the formula was the same. The cells of the fused and screened positive wells were prepared into cell suspensions, and 100ul of the cell suspensions were taken and added into each well of the first row, uniformly mixed. Then 100µl of the cell suspensions of the 1st row were added into the 2nd row, uniformly mixed, and 100µl of the cell suspensions were added into the next row; the above steps are repeated, the 96-well plate was kept to stand for 30min, and the cells were observed and counted under a microscope. A volume corresponding to 100 cells was added into 20ml of medium, uniformly mixed, and then plated at 200µl per well. One week later, the wells were observed under a microscope to identify and mark the monoclonal wells. When the confluence of cells in eah well reaches more than 50%, the above-mentioned FACS screening method and blocking experiment are used for detection, and the target positive wells are selected for in vitro screening. The affinity of the obtained hybridoma cell supernatant to the antigen was detected by biofilm thin layer interferometry (ForteBio).

### Hybridomas sequencing

The positive clones obtained by screening were subjected to hybridoma sequencing to obtain the antibody variable region sequence.

RNA extraction: RNA extraction kit (Macherey-Nagel, Cat #740984.250) was used to extract RNA from hybridoma cells. About 5×10⁶ fresh cultured cells were taken, centrifuged at 300 g for 5 min and the supernatant was removed, and 500 µl of LBP buffer was added to the precipitate and mixed until clear. The mixture was added to a DNA removal tube, centrifuged at 11000 rpm for 1 min, and the flow-through solution was collected. 100ul LBS was added into the flow-through liquid and uniformly mixed. The clear solution was added to the RNA collection tube, centrifuged at 11000rpm for 1 min to remove the liquid, added with 200 µl WB1, and centrifuged at 11000rpm for 1 min to remove the liquid. 600ul of WB2 was added to the RNA collection tubes and centrifuged at 11000rpm for 1 min to remove liquid. 250ul WB2 was added to the RNA collection tube, centrifuged at 11000rpm for 2min, the liquid was discarded, then the RNA collection tube was left idle and centrifuged for 1min to remove the liquid completely. The RNA column was uncapped and transferred to a new 1.5 mL EP tube. After ethanol was evaporated for 2 min, 50 µl DEPC treated ddH₂O was added, left to stand for 2 min and centrifuged at 11,000 g for 1 min, and the eluate was collected. The RNA concentration was determined.

Reverse transcription PCR: cDNA was obtained by reverse transcription using PrimeScript^{™} RT reagent Kit (Takara) in the following reaction system: 5×PrimeScript Buffer 4ul, 1× PrimeScript RT Enzyme Mix 1ul, Oligo dT Primer (50µM) 1ul, Total RNA 8ul, ddH₂O 6 ul. The reaction procedure was as follows: 37°C 15 min, 85°C 5s, 4°C hold. The RT-PCR product cDNA was obtained.

PCR amplification of VH/VL sequences: two cDNA portions were taken to amplify the variable regions of the heavy chain and the light chain respectively. The reaction system is as follows: ExTag(ExTaq Hot Start Version, Takara) 0.5ul, 10×ExTaq buffer 3ul, dNTP 4ul, Forward primer 2ul, Reverse primer 2ul, template (cDNA) 1ul, ddH₂O 17.5ul. The reaction procedure was as follows: step 1: 94°C 5min; step 2: 94°C 30s; step 3: 55°C 30s; step 4: 72°C 40s; step 5: 72°C 10min. Wherein the steps 2 to 4 were circulated for 30 times. The PCR product was ligated to the T-vector using the Mighty TA-cloning Kit (Takara).

**Transformation of the cells:** TOP10 competent cells (TIANGEN BIOTECH (BEIJING) Co.,LTD.) was taken from -80°C, thawed on ice, 5 µl of the ligation product obtained above was added to the thawed TOP10 competent cells, mixed well and incubated on ice for 30 min, then heat shocked at 42°C for 90 s, rapidly cool down on ice for 2 min. 900 µl LB medium (Sangon Biotech (Shanghai) Co., Ltd.) was added to the EP tube and cultured at 37°C, 220 rpm on a shaker for 1 h. After centrifugation at 3000 g for 2 min and removing 800 µl of supernatant, the bacteria were resuspended with the remaining medium and plated on the plate with ampicillin. After overnight incubation at 37°C, colonies were picked for sequencing and the sequencing results were analyzed and aligned using MEGA7 software.

### Construction of chimeric antibodies

The heavy chain and light chain sequences of the anti-OX40L antibody produced by the above hybridoma cells are obtained by utilizing molecular biological technique. The light chain and heavy chain variable region gene fragments of the heavy chain and light chain sequences were respectively ligated into a pcDNA3.1 vector through homologous recombinase, wherein IgG4 subtype was selected as the constant region (the heavy chain constant region: comprising the S228P mutation, SEQ ID NO: 21; the light chain constant region: SEQ ID NO: 22), and expression plasmids of the light chain and heavy chain antibodies were obtained. Then, the light chain plasmid and the heavy chain plasmid of the same antibody were mixed at a molar ratio of 1:1 to transfect 293F cells with Polyethyleneimine (PEI) (Polysciences, Cat #23966). After 5-7 days of culture, when the cell viability was less than 60%, cell culture supernatants were collected and purified with Protein A affinity columns to get monoclonal antibodies, then obtaining the human-mouse chimeric antibodies.

Screening for chimeric antibody: the anti-OX40L antibody which can simultaneously bind to OX40L and block OX40-OX40L interaction was obtained by in-vitro experimental screening adopting flow cytometry and luciferase reporter gene method.

### Antibody humanization

The chimeric antibody obtained in the above Example was humanized according to a conventional method. The CDR sequence, light chain variable region sequence and heavy chain variable region sequence of the humanized antibody, and the amino acid sequences of the light chain and the heavy chain are referred to the sequence list.

### Expression and purification of humanized antibodiy

HEK293 cells (Invitrogen) were passaged according to the desired transfection volume and the cell density was adjusted to 1.5×10⁶ cells/ml the day before transfection. The cell density at the day of transfection was approximately 3×10⁶ cells/ml. 1/10 (v/v) final volume of Opti-MEM medium (Gibco Cat# 31985-070) was taken as transfection buffer, added with the expression plasmid pcDNA3.1 vector containing the heavy and light chains of the humanized antibody, respectively, which contained nucleic acids encoding the heavy and light chains of the HZ18 and HZ22 antibodies, mixed uniformly and filtered with a 0.22 µm filter for use. Appropriate Polyethyleneimine (PEI) (Polysciences, 23966) was added into the plasmid in the last step (the mass ratio of the plasmid to the PEI is 1:3), mixed uniformly, and incubated at room temperature for 10 min to obtain a DNA/PEI mixture. The DNA/PEI mixture was poured gently into HEK293 cells and mixed well, after 24h of culture at 37°C under 8% CO₂, supplemented with VPA (Sigma, Cat # P4543-100G) at a final concentration of 2 mM and 2% (v/v) Feed solution (1 g/L Phytone Peptone + 1g/L Difco Select Phytone) and the culture was continued for 6 days.

After the cell culture, the cell culture fluid was centrifuged at 13000 rpm for 20 min, the supernatant was collected and purified with a pre-packed column Hitrap Mabselect Sure (GE, 11-0034-95) according to the manufacturer's instructions, and the concentration was determined. 100 µg of the purified protein was adjusted to a concentration of 1 mg/mL, and the purity of the protein was measured using a gel filtration chromatography column SW3000 (TOSOH Cat # 18675). The result indicated that an antibody having a high purity was obtained.

### Affinity assay of humanized antibody

The affinity of the humanized antibody for the antigen (human and cynomolgus OX40L) was determined using ForteBio assay and expressed as the equilibrium dissociation constant (KD). The results are shown in Table 1.

**Table 1. Affinity constants (M) for antigen-antibody binding detected by ForteBio**

| Anti-OX40L antibodies | Human OX40L |
|---|---|
| Hz18 | 3.07E-10 |
| Hz22 | 3.79E-10 |

In the present application, the inventors of the present invention screened antibodies with higher affinity to OX40L, and finally obtained two humanized antibodies HZ18 and HZ22 by humanization. In this example, their blocking effects on OX40L at the molecular and cellular levels were tested. Meanwhile, in the co-culture system of CHO-OX40L overexpressing cells and Jurkat-OX40- NFκB-Luc reporter cells, the addition of the anti-OX40L antibody of the present invention blocked the binding of OX40L on the surface of CHO cells and OX40 on the surface of Jurkat-OX40- NFκB-Luc reporter, thereby inhibiting the activation of NFκB and luciferase production downstream of Jurkat cells, and reducing the fluorescence signal of the system.

### ELISA detection of antibody blocking OX40/OX40L binding at protein level

The experimental steps are as follows: the OX40L recombinant protein was diluted to a concentration of 1µg/ml using Citrate-buffered saline, added to a 96-well ELISA plate at 100 µl per well, and incubated overnight at 4°C. The next day, the coated ELISA plate was washed three times with PBST buffer (1 ×PBS, 0.05% Tween), patted to dry. The blocking solution (1 ×PBS, 0.05% Tween, 1% BSA) was added to the 96-well ELISA plate and incubated at room temperature for 2 h, then washed three times with PBST buffer and patted to dry. The OX40-Avi was diluted to 1 µg/ml with the blocking solution, while the antibody to be tested was diluted with the blocking solution. 50 ul of diluted OX40-Avi and 50 ul of diluted antibody to be tested were added to a 96-well ELISA plate respectively, incubated at room temperature for 2h. The plate was washed three times with PBST buffer and patted to dry. The Streptavidin-HRP was diluted with blocking solution (1: 3000), added to a 96-well ELISA plate at 100 µl per well, and incubated at room temperature in the dark for 1h. The plate was washed three times with PBST buffer and patted to dry. 100 µl of TMB developing solution was added to the 96-well ELISA plate for color developing, and the development was stopped using a stop solution. OD450 absorbance was read in a microplate reader.

### Detection by reporter gene system for blocking of OX40 signal activation by antibody

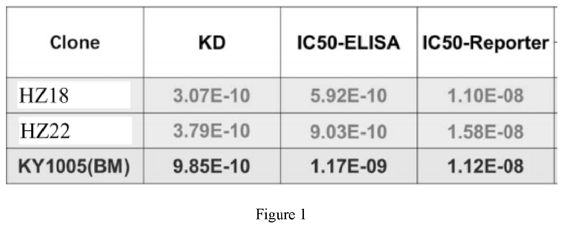

The experimental steps are as follows: OX40L overexpressing cells (CHO-OX40L) and OX40 overexpressing reporter cells (Jurkat-OX40-NFκB-Luc) were prepared in good growth conditions. The cells were counted. CHO-OX40L was adjusted to 5.5×10^4/ml and Jurkat-OX40-NF κ B-Luc was diluted to 2.2×10^6/ml using 1640 complete medium, while the antibody was diluted using 1640 complete medium. 45 µl CHO-OX40L and 45 µl Jurkat-OX40-NFκB-Luc cells were added to a 96-well white bottom plate, along with 10 µl of antibody. The 96-well white bottom plate was placed in a 37°C cell incubator and incubated for 6 h. After the incubation, 100 µl of Bio-Glo Reagent was added to each well, and reacting at room temperature in the dark for 10 min. The plate was placed on a multifunctional microplate reader to read fluorescence value. Figure. 1 shows the results of the experiment. Among them, an antibody KY1005 (US10654935B2).

### Example 2: Inhibition on primary T cell activation by anti-OX40L antibodies

The effect of anti-OX40L antibodies on T cell activation was detected in a co-culture system of CHO-OX40L overexpressing cells and primary T cells, and the inhibitory effect was reflected by measuring the level of IL-2 secretion in the culture system.

The experimental procedure was as follows: OKT3 was diluted to 1µg/ml and added to a 96-well U-bottom plate at 100 µl per well for coating at 4°C overnight. The following day, the liquid in the 96-well U-plate was removed, and the 96-well U-bottom plate was washed three times with 1×PBS and patted to dry. Pan T cells in PBMC were isolated using a Pan-T cell isolation kit (Miltenyi Biotec) according to the protocol of the instructions, adjusted to a cell density of 1×10⁶/ml using 1640 complete medium (RPMI medium 1640+5%FBS), and added with anti-human CD28. CHO-OX40L cells were diluted to 0.8×10⁵/ml using 1640 complete medium, and the antibody to be tested was diluted using 1640 complete medium. 100 µl Pan T cells, 50 µl CHO-OX40L and 50 µl antibody to be tested were added to patted-dry 96-well U-bottom plate. The 96-well U-bottom plate was placed in a 37°C cell incubator and incubated for 3 days. After three days, the 96-well U-bottom plate was taken out, centrifuged at 500×g for 10 min, and the culture supernatant was pipetted. The IL-2 content in the supernatant was detected using IL-2 ELISA (Human IL-2 DuoSet ELISA, R&D) to indicate the activation of T cells.

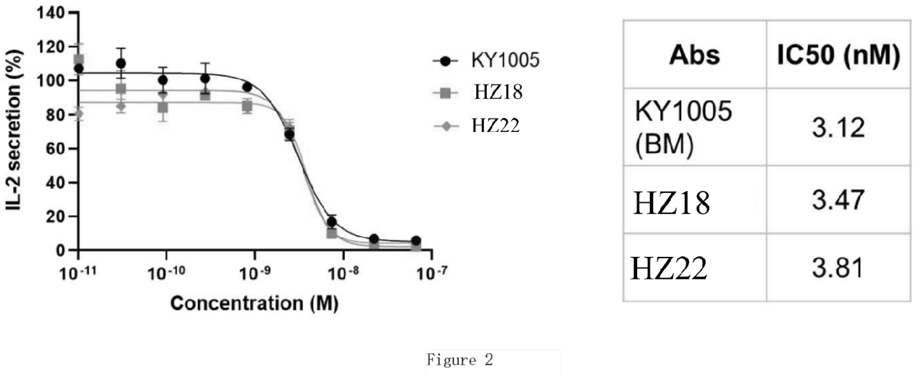

Figure. 2 shows the results of the experiment. It can be seen from Figure 2 that the blocking antibody to OX40L can significantly inhibit the activation level of T cells in vitro.

### Example 3: Effect of anti-OX40L antibodies on DCs-induced T cell differentiation to Th2

It has been shown in the existing research that Thymic stromal lymphopoietin (TSLP) can induce the expression of OX40L on dendritic cells, and further promote T cells to differentiate towards Th2 during antigen presentation, and secrete Th2 cell-related factors of IL-4, IL-5 and IL-13.

In this experiment, isolated primary DC cells were stimulated with recombinant hTSLP (Sino Biological), followed by co-culture of DCs with isolated naive CD4 T cells for 6 days, and then anti-CD3/anti-CD28 antibodies were added to further activate T cells. The anti-OX40L antibody or isotype-control was added to the whole culture system, and the inhibitory effect of the antibody was evaluated by detecting the secretion levels of IL-5 and IL-13.

### The experimental steps:

mDCs were isolated using the EasySep Human Myeloid DC Enrichment Kit (Stemcell):
The PBMC was taken and thawed quickly, and washed twice with 1× PBS; resuspended to 5×10⁷ cells/ml using MACS Buffer. The system was added with FcR blocker at 15 µl/ml, Myeloid DC Enrichment Crocktail Component A at 50 µl/ml and DC Enrichment Crocktail Component B at 50 µl/ml, uniformly mixed, and incubated at room temperature in the dark for 30min. The separated magnetic beads was taken, shaken and mixed well. The system was added with the magnetic beads at 100 µl/ml, mixed uniformly and incubated at room temperature in the dark for 10min. The volume of the system was added to 2.5 ml with MACS Buffer and transferred to a flow tube. The flow tube was placed on a magnetic frame, and waited for 5min at room temperature. The liquid was pipepted into a new flow tube, which was continuously placed in a magnetic field, and waited for 5 min at room temperature. The liquid was pipetted into a 15 ml tube, centrifuged at 500× g for 10 min. The mDC was resuspended using X-VIVO 15, and centrifuged at 500×g for 10 min. The mDC was resuspended using X-VIVO 15 (Lonza) and the cell density was adjusted to 1×10^6 cells/ml. Recombinant hTSLP was added to the mDC suspension at a final concentration of 20 ng/ml and cells were inoculated into an ultra-low attachment 96-well u-bottom plate at 200 µl per well. The mDC cells in the ultra-low attachment 96-well u-bottom plate were cultured at 37°C for 2 days.

The mDC cells were taken out of the incubator, centrifuged at 500× g for 10 min to remove supernatant, resuspended with fresh X-VIVO 15 medium, centrifuged at 500×g for 10 min. The cells were continuously resuspended using fresh X-VIVO 15 medium, centrifuged at 500×g for 10 min to remove supernatant. Concentration of the mDC was adjusted to 160000 cells/ml using X-VIVO 15.

The EasySep Human Naive CD4+T Cell Isolation Kit (Stemcell) was used to isolate naive CD4+T cells: the PBMC was taken and thawed quickly, and washed twice with 1× PBS; resuspended to 5×10⁷ cells/ml using MACS Buffer; added with Biotinylated Anti-CD45RO Antibody at 50 µl/ml and Isolation Cocktail at 50 µl/ml, mixed uniformly, and standed at room temperature for 5 min. The separated magnetic beads was taken out, shaked and mixed well. The magnetic beads was added into the system at 50 µl/ml, mixed uniformly , and standed at room temperature for 5 min. The volume of the system was added to 2.5 ml with MACS Buffer and transferred to a flow tube which was placed on a magnetic frame, and waited for 5min at room temperature. The liquid was pippeted into a new flow tube which was continuously placed in a magnetic field, and waited for 5 min at room temperature. The liquid was pippeted into a new flow tube which was continuously placed in a magnetic field, and waited for 5 min at room temperature. The liquid was pippeted into a 15 ml tube, centrifuged at 500× g for 10 min. T cells was resuspended using X-VIVO 15, and centrifuged at 500×g for 10 min. The naive CD4+ T cells was resuspended using X-VIVO 15 and the cell density was adjusted to 400000 cells/ml.

50 µl of diluted mDC, 100 µl of naive CD4+T cells and 50 µl of antibody were added to an ultra-low attachment 96-well u-bottom plate, and the ultra-low attachment 96-well u-bottom plate was incubated in a 37°C, 5% CO₂ cell incubator for 4 days. After the co-incubation system was cultured for 4 days, the ultra-low attachment 96-well u-bottom plate was taken out and centrifuged at 500×g for 10 min. 140 µl of supernatant was pipetted and discarded. The 4× antibody was diluted as above and 120 µl of fresh X-VIVO 15 medium and 40 µl 4× antibody were added to the ultra-low attachment 96-well u-bottom plate. The cells in the ultra-low attachment 96-well u-bottom plate were blown evenly. The ultra-low attachment 96-well u-bottom plate was placed in a 37°C, 5% CO₂ cell incubator and incubated continuously for 3 days. The ultra-low attachment 96-well u-bottom plate was taken out and centrifuged at 500×g for 10 min. 150µl of supernatant was pipetted and discarded; Anti-Human CD28 was diluted to 3.33 µg/ml (1.67×) using X-VIVO 15 and added at 120 µl per well to U-bottom plate coated with OKT3 the previous day. The 4×antibody to be tested was diluted as above and added at 40 µl per well to U-bottom plate coated the previous day. The cells which are centrifuged to the bottom of the ultra-low attachment 96-well u-bottom plate were uniformly blown, and completely transferred to the 96-well u-bottom plate coated the previous day; placed into an 37°C incubator for 1 day. The 96-well U-plate were taken from the incubator, centrifuged at 500×g for 10 min. 140 µl of supernatant was pipetted into a new V-bottom plate and placed at -80°C, for use in the ELISA experiments in which cytokines were dectected using Human IL-5 DuoSet ELISA and Human IL-13 ELISA BASIC kit (HRP).

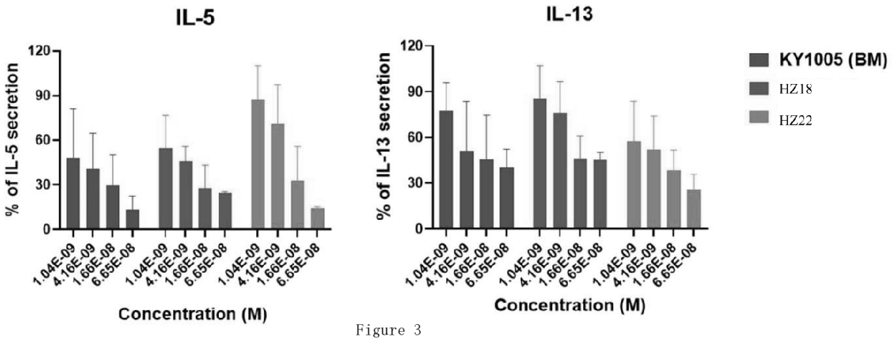

Figure 3 shows the results of the experiment. It can be seen from Figure 3 that blocking antibody to OX40L can significantly inhibit Th2 inflammatory responses.

### Example 4: Effect of anti-OX40L antibody in a mouse model of graft-versus-host disease (GvHD)

### Part 1: GvHD animal experiment process and disease scoring

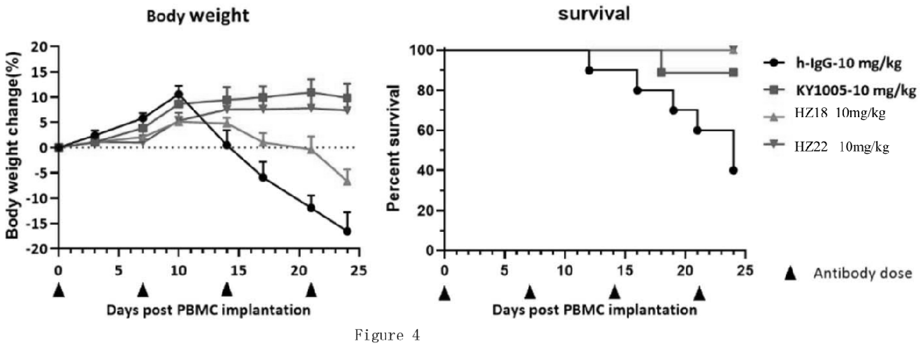

NOG mice were injected intravenously with PBMC (1*10^7/mouse) to establish a GVHD model. Immediately, the mice were randomly divided into 4 groups, with 9 mice in each group. Administration was performed once a week for a total of four times, namely D0, D7, D14, and D21, respectively. The body weight of the mice was recorded 2 times a week, and the survival of the mice was observed. The weight change graph and survival curve were prepared, as shown in Figure 4. Blood was collected on D14 for flow cytometry detection. In the experiment, HZ22 can effectively reduce the weight loss caused by mouse GVHD and obviously reduce the death rate of the mouse; HZ18 can significantly reduce the death rate of mice.

### PBMC information

Manufacturer: AllCellS Catalog number: FPB005F-C Batch number: 3024702
Mouse information
Strain: NOG Gender: Female Source: Beijing Vital River Laboratory Animal Technology Co., Ltd. Certificate No.: 110011211111216336

### Antibody information

| Name | Manufactuer | Catalog number | Batch number |
|---|---|---|---|
| H-IgG | Equitech-Bio | SLH56-0001 | 161206-0656 |

### Part 2: Flow cytometry detection-related marker

In this experiment, blood was collected on the 14th day of modeling, and the number of CD4 T cells in the mouse blood was analyzed by flow cytometry. And the proportion of Granzyme B positive cells in CD4 T cells was detected. At the same time, the amount of IFN-γ and IL-6 in the serum was detected by ELISA. As shown in Figue 5, it can be seen from Figue 5 that the use of a blocking antibody to OX40L can inhibit the secretion of IFNγ and IL-6 in peripheral blood of GvHD mice, and also inhibit the activation of T cells.

### Summary of sequence:

| Antibody Name | SEQ ID NO | HZ18 | SEQ ID NO | HZ22 |
|---|---|---|---|---|
| HCDR1 | SEQ ID NO:1 | GYSFTGYRIH | SEQ ID NO:11 | GYAVTGDYAWS |
| HCDR2 | SEQ ID NO:2 | YVDPYDDKTDYSQKSKG | SEQ ID NO:12 | YISSSAGTNYNPSLKS |
| HCDR3 | SEQ ID NO:3 | EGFAY | SEQ ID NO:13 | DLYYGGRNQWYFDV |
| VH | SEQ ID NO:4 | EVQLVQSGAEVKKPGAT VKISCKVSGYSFTGYRIH WVQQAPGKGLEWMGYV DPYDDKTDYSQKSKGRV TITADTSTDTAYMELSSLR SEDTAVYYCAREGFAYW GQGTLVTVSS | SEQ ID NO:14 | QVQLQESGPGLVKPSQTLSLTCTVSGYAV TGDYAWSWIRQPPGKRLEWIGYISSSAGT NYNPSLKSRVTISVDTSKNQFSLKLSSVTA ADTAVYYCARDLYYGGRNQWYFDVWGQ GTLVTVSS |
| LCDR1 | SEQ ID NO:5 | TSSSSLRYMH | SEQ ID NO:15 | SATSSIIYMH |
| LCDR2 | SEQ ID NO:6 | NTFDLAS | SEQ ID NO:16 | DTSKLPS |
| LCDR3 | SEQ ID NO:7 | QQRTTYPPT | SEQ ID NO:17 | HQRSSFPFT |
| VL | SEQ ID NO:8 | DIQMTQSPSSLSASVGDR VTITCTSSSSLRYMHWYQ QKPGKAPKLLIYNTFDLA SGVPSRFSGSGSGTDFTLT ISSLQPEDFATYYCQQRTT YPPTFGQGTKLEIK | SEQ ID NO:18 | DIQMTQSPSSLSASVGDRVTITCSATSSIIY MHWYQQKPGKAPKLLIYDTSKLPSGVPSR FSGSGSGTDFTLTISSLQPEDFATYYCHQRS SFPFTFGGGTKVEIK |
| Heavy chain | SEQ ID NO:9 | EVQLVQSGAEVKKPGAT VKISCKVSGYSFTGYRIH WVQQAPGKGLEWMGYV DPYDDKTDYSQKSKGRV TITADTSTDTAYMELSSLR SEDTAVYYCAREGFAYW GQGTLVTVSSASTKGPSV FPLAPCSRSTSESTAALGC LVKDYFPEPVTVSWNSGA LTSGVHTFPAVLQSSGLY SLSSVVTVPSSSLGTKTYT CNVDHKPSNTKVDKRVE SKYGPPCPPCPAPEFLGGP SVFLFPPKPKDTLMISRTP EVTCVVVDVSQEDPEVQF NWYVDGVEVHNAKTKPR EEQFNSTYRVVSVLTVLH QDWLNGKEYKCKVSNKG LPSSIEKTISKAKGQPREP QVYTLPPSQEEMTKNQVS LTCLVKGFYPSDIAVEWE | SEQ ID NO:19 | QVQLQESGPGLVKPSQTLSLTCTVSGYAV TGDYAWSWIRQPPGKRLEWIGYISSSAGT NYNPSLKSRVTISVDTSKNQFSLKLSSVTA ADTAVYYCARDLYYGGRNQWYFDVWGQ GTLVTVSSASTKGPSVFPLAPCSRSTSESTA ALGCLVKDYFPEPVTVSWNSGALTSGVHT FPAVLQSSGLYSLSSVVTVPSSSLGTKTYT CNVDHKPSNTKVDKRVESKYGPPCPPCPA PEFLGGPSVFLFPPKPKDTLMISRTPEVTCV VVDVSQEDPEVQFNWYVDGVEVHNAKTK PREEQFNSTYRVVSVLTVLHQDWLNGKEY KCKVSNKGLPSSIEKTISKAKGQPREPQVY TLPPSQEEMTKNQVSLTCLVKGFYPSDIAV EWESNGQPENNYKTTPPVLDSDGSFFLYSR LTVDKSRWQEGNVFSCSVMHEALHNHYT QKSLSLSLGK |
| | | SNGQPENNYKTTPPVLDS DGSFFLYSRLTVDKSRWQ EGNVFSCSVMHEALHNH YTQKSLSLSLGK | | |
| Light chain | SEQ ID NO:10 | DIQMTQSPSSLSASVGDR VTITCTSSSSLRYMHWYQ QKPGKAPKLLIYNTFDLA SGVPSRFSGSGSGTDFTLT ISSLQPEDFATYYCQQRTT YPPTFGQGTKLEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQG LSSPVTKSFNRGEC | SEQ ID NO:20 | DIQMTQSPSSLSASVGDRVTITCSATSSIIY MHWYQQKPGKAPKLLIYDTSKLPSGVPSR FSGSGSGTDFTLTISSLQPEDFATYYCHQRS SFPFTFGGGTKVEIKRTVAAPSVFIFPPSDE QLKSGTASVVCLLNNFYPREAKVQWKVD NALQSGNSQESVTEQDSKDSTYSLSSTLTL SKADYEKHKVYACEVTHQGLSSPVTKSFN RGEC |
| Heavychain constant region ( having S228P mutation) | SEQ ID NO:21 | ASTKGPSVFPLAPCSRSTS ESTAALGCLVKDYFPEPV TVSWNSGALTSGVHTFPA VLQSSGLYSLSSVVTVPSS SLGTKTYTCNVDHKPSNT KVDKRVESKYGPPCPPCP APEFLGGPSVFLFPPKPKD TLMISRTPEVTCVVVDVS QEDPEVQFNWYVDGVEV HNAKTKPREEQFNSTYRV VSVLTVLHQDWLNGKEY KCKVSNKGLPSSIEKTISK AKGQPREPQVYTLPPSQE EMTKNQVSLTCLVKGFYP SDIAVEWESNGQPENNYK TTPPVLDSDGSFFLYSRLT VDKSRWQEGNVFSCSVM HEALHNHYTQKSLSLSLG K | SEQ ID NO:21 | |
| Light chan constant region | SEQ ID NO:22 | | SEQ ID NO:22 | |
| VH DNA | SEQ ID NO:23 | GAGGTGCAGCTGGTGCA GAGCGGAGCAGAGGTGA AGAAGCCAGGGGCCACA GTGAAGATAAGCTGTAA GGTGAGCGGATATAGCT TTACAGGGTACAGAATT CACTGGGTGCAGCAGGC ACCCGGCAAAGGACTGG AGTGGATGGGATATGTG GACCCCTACGATGACAA GACCGACTACAGCCAGA AAAGCAAGGGCAGAGTG ACCATTACCGCAGACAC AAGCACCGACACAGCCT ACATGGAGCTGAGCAGC CTGAGAAGCGAGGATAC CGCCGTGTACTACTGCG CCAGAGAAGGATTCGCC TACTGGGGGCAAGGGAC CCTGGTGACCGTGAGCA GC | SEQ ID NO:25 | CAGGTCCAACTCCAAGAGAGCGGCCCCG GCCTCGTCAAACCCAGCCAAACACTCTC CCTCACCTGCACAGTCAGCGGCTACGCC GTCACAGGAGACTACGCCTGGAGCTGGA TCAGACAACCCCCCGGCAAAAGACTGGA GTGGATCGGCTATATCAGCAGCAGCGCC GGCACCAACTACAACCCCAGCCTGAAAA GCAGAGTGACAATAAGCGTGGACACTTC TAAGAACCAGTTCAGCCTCAAACTGAGC TCCGTGACCGCCGCAGACACCGCCGTGT ACTACTGCGCCCGAGACCTCTACTACGG CGGAAGAAACCAGTGGTACTTCGATGTG TGGGGCCAAGGCACCCTGGTGACCGTGA GCAGC |
| VL DNA | SEQ ID NO:24 | GACATTCAGATGACCCA GAGCCCCAGCAGCCTGA GCGCCAGCGTGGGAGAC AGAGTGACCATAACCTG CACCTCCAGCAGCAGCC TGAGATACATGCACTGG TACCAGCAGAAACCCGG GAAGGCCCCCAAACTGC TCATCTACAACACCTTCG ACCTGGCCAGCGGCGTG CCCAGCAGATTCTCCGG CAGCGGAAGCGGAACAG ACTTCACCCTGACTATCA GCAGCCTCCAACCCGAA GACTTCGCAACTTACTAC TGCCAACAGCGGACCAC CTATCCACCCACCTTCGG CCAGGGCACCAAGCTGG AAATCAAA | SEQ ID NO:26 | GACATTCAGATGACCCAGAGCCCCAGCA GCCTGAGCGCCAGCGTGGGAGACAGAGT GACCATAACCTGCAGCGCCACATCCAGC ATAATATATATGCACTGGTATCAGCAAA AGCCCGGCAAGGCCCCCAAACTGCTCAT CTACGACACCAGCAAGCTGCCCAGCGGC GTGCCTAGCCGCTTCTCCGGAAGCGGCT CCGGAACCGACTTCACCCTCACTATCAG CAGCCTGCAACCCGAAGACTTCGCCACC TACTACTGCCACCAAAGAAGCTCCTTCC CCTTCACATTCGGCGGTGGCACCAAAGT GGAGATCAAG |
| Human IgG4 Fc(having S228P mutation) | SEQ ID NO:27 | PPCPPCPAPEFLGGPSVFLFP [?]KPKDTLMISRTPEVTCVVVDVSQEDPEVQFNWYVDGVEVHNA KTKPREEQFNSTYRVVSVLTVLHQDWLNGKEYKCKVSNKGLPSSIEKTISKAKGQPREPQVYTL PPSQEEMTKNQVSLTCLVKGFYPSDIAVEWESNGQPENNYKTTPPVLDSDGSFFLYSRLTVDKSR WQEGNVFSCSVMHEALHNHYTQKSLSLSLGK | | |
| Human IgG4 Fc (wild type) | SEQ ID NO:28 | PPCPSCPAPEFLGGPSVFLFPPKPKDTLMISRTPEVTCVVVDVSQEDPEVQFNWYVDGVEVHNA KTKPREEQFNSTYRVVSVLTVLHQDWLNGKEYKCKVSNKGLPSSIEKTISKAKGQPREPQVYTL PPSQEEMTKNQVSLTCLVKGFYPSDIAVEWESNGQPENNYKTTPPVLDSDGSFFLYSRLTVDKSR WQEGNVFSCSVMHEALHNHYTQKSLSLSLGK | | |
| Human IgG4 heavy chain constant ( without mutation) | SEQ ID NO:29 | ASTKGPSVFPLAPCSRSTSESTAALGCLVKDYFPEPVTVSWNSGALTSGVHTFPAVLQSSGLYSL SSVVTVPSSSLGTKTYTCNVDHKPSNTKVDKRVESKYGPPCPSCPAPEFLGGPSVFLFPPKPKDT LMISRTPEVTCVVVDVSQEDPEVQFNWYVDGVEVHNAKTKPREEQFNSTYRVVSVLTVLHQD WLNGKEYKCKVSNKGLPSSIEKTISKAKGQPREPQVYTLPPSQEEMTKNQVSLTCLVKGFYPSD IAVEWESNGQPENNYKTTPPVLDSDGSFFLYSRLTVDKSRWQEGNVFSCSVMHEALHNHYTQK SLSLSLGK | | |
| Human OX40L | SEQ ID NO:30 | MERVQPLEENVGNAARPRFERNKLLLVASVIQGLGLLLCFTYICLHFSALQVSHRYPRIQSIKVQ FTEYKKEKGFILTSQKEDEIMKVQNNSVIINCDGFYLISLKGYFSQEVNISLHYQKDEEPLFQLK KVRSVNSLMVASLTYKDKVYLNVTTDNTSLDDFHVNGGELILIHQNPGEFCVL | | |

## Claims

1. An anti-OX40L antibody or an antigen-binding fragment thereof, the antibody or an antigen-binding fragment thereof comprising:
(i) three complementarity-determining regions HCDR1, HCDR2, and HCDR3 contained in the VH as set forth in SEQ ID NO:4, and the three complementarity-determining regions LCDR1, LCDR2, and LCDR3 contained in the VL as set forth in SEQ ID NO:8; or
(ii) the three complementarity-determining regions HCDR1, HCDR2, and HCDR3 contained in the VH as set forth in SEQ ID NO:14, and the three complementarity-determining regions LCDR1, LCDR2, and LCDR3 contained in the VL as set forth in SEQ ID NO:18.

2. An anti-OX40L antibody or an antigen-binding fragment thereof, comprising a first heavy chain complementary determining region (HCDR1), a second heavy chain complementary determining region (HCDR2), a third heavy chain complementary determining region (HCDR3), and a first light chain complementary determining region (LCDR1), a second light chain complementary determining region (LCDR2), and a third light chain complementary determining region (LCDR3), wherein
(i) the HCDR1, HCDR2, HCDR3, and LCDR1, LCDR2, and LCDR3 comprise the amino acid sequences set forth in SEQ ID NO:1, SEQ ID NO:2, SEQ ID NO:3, SEQ ID NO:5, SEQ ID NO:6, and SEQ ID NO:7 respectively, or consist of the amino acid sequences set forth in SEQ ID NO:1, SEQ ID NO:2, SEQ ID NO:3, SEQ ID NO:5, SEQ ID NO:6, and SEQ ID NO:7 respectively; or
(ii) the HCDR1, HCDR2, HCDR3, and LCDR1, LCDR2, and LCDR3 comprise the amino acid sequences set forth in SEQ ID NO:11, SEQ ID NO:12, SEQ ID NO:13, SEQ ID NO:15, SEQ ID NO:16, and SEQ ID NO:17 respectively, or consist of the amino acid sequences set forth in SEQ ID NO:11, SEQ ID NO:12, SEQ ID NO:13, SEQ ID NO:15, SEQ ID NO:16, and SEQ ID NO:17 respectively.

3. The anti-OX40L antibody or antigen-binding fragment according to claim 1 or 2, which comprises a heavy chain variable region (VH), wherein said heavy chain variable region comprises an amino acid sequence having at least 90% identity to the amino acid sequence as set forth in SEQ ID NO:4 or 14, or consists of said sequence or comprises the amino acid sequence as set forth in SEQ ID NO:4 or 14, or consists of said sequence; or which comprises a light chain variable region (VL), wherein said light chain variable region comprises an amino acid sequence having at least 90% identity to the amino acid sequence as set forth in SEQ ID NO:8 or 18, or consists of said sequence or comprises the amino acid sequence as set forth in SEQ ID NO:8 or 18, or consists of said sequence.

4. An anti-OX40L antibody or an antigen-binding fragment thereof, comprising a heavy chain variable region and a light chain variable region, wherein
(i) the heavy chain variable region comprises or consists of the amino acid sequence set forth in SEQ ID NO: 4, or an amino acid sequence having at least 90%, 91%, 92%, 93%, 94%, 95%, 96%, 97%, 98% or 99% identity thereto; and the light chain variable region comprises or consists of the amino acid sequence set forth in SEQ ID NO: 8, or an amino acid sequence having at least 90%, 91%, 92%, 93%, 94%, 95%, 96%, 97%, 98% or 99% identity thereto;
(ii) the heavy chain variable region comprises or consists of the amino acid sequence set forth in SEQ ID NO: 4, and the light chain variable region comprises or consists of the amino acid sequence set forth in SEQ ID NO: 8;
(iii) the heavy chain variable region comprises or consists of the amino acid sequence set forth in SEQ ID NO: 14, or an amino acid sequence having at least 90%, 91%, 92%, 93%, 94%, 95%, 96%, 97%, 98% or 99% identity thereto; and the light chain variable region comprises or consists of the amino acid sequence set forth in SEQ ID NO: 18, or an amino acid sequence having at least 90%, 91%, 92%, 93%, 94%, 95%, 96%, 97%, 98% or 99% identity thereto; or
(iv) the heavy chain variable region comprises or consists of the amino acid sequence set forth in SEQ ID NO: 14, and the light chain variable region comprises or consists of the amino acid sequence set forth in SEQ ID NO: 18.

5. The anti-OX40L antibody or antigen-binding fragment according to claim 1 or 2, which comprises a heavy chain variable region and a light chain variable region, wherein
(i) the heavy chain variable region comprises or consists of the amino acid sequence set forth in SEQ ID NO: 4, and the light chain variable region comprises or consists of the amino acid sequence set forth in SEQ ID NO: 8; or
(ii) the heavy chain variable region comprises or consists of the amino acid sequence set forth in SEQ ID NO: 14, and the light chain variable region comprises or consists of the amino acid sequence set forth in SEQ ID NO: 18.

6. The anti-OX40L antibody or antigen-binding fragment according to any one of claims 1 to 5, which comprises an Fc region.

7. The anti-OX40L antibody or antigen-binding fragment according to claim 6, wherein said Fc region is an Fc from a human IgG, for example, an Fc from human IgG1, an Fc from human IgG2, an Fc from human IgG3, or an Fc from human IgG4.

8. The anti-OX40L antibody or antigen-binding fragment according to claim 7, wherein said Fc region comprises a mutation that enhances the stability of the Fc region or reduces antibody chain exchange in vivo.

9. The anti-OX40L antibody or antigen-binding fragment according to claim 8, wherein said mutation is an S228P mutation.

10. The anti-OX40L antibody or antigen-binding fragment according to any one of claims 6 to 9, wherein said Fc region
(i) comprises or consists of the amino acid sequence of SEQ ID NO:27 or 28;
(ii) comprises or consists of an amino acid sequence having at least 90% identity, for example, 95%, 96%, 97%, 99% or higher identity, to the amino acid sequence set forth in SEQ ID NO:28; or
(iii) comprises an amino acid sequence having at least 90% identity, for example, 95%, 96%, 97%, 99% or higher identity, to the amino acid sequence set forth in SEQ ID NO:27 and comprising an S228P mutation.

11. The anti-OX40L antibody or antigen-binding fragment according to any one of claims 1 to 10, which comprises a heavy chain constant region, said heavy chain constant region being from the constant region of IgG1, IgG2, IgG3 or IgG4, preferably, said heavy chain constant region
(i) comprises or consists of the amino acid sequence selected from SEQ ID NO:21 or 29;
(ii) comprises an amino acid sequence having at least 85%, 90%, 91%, 92%, 93%, 94%, 95%, 96%, 97%, 98% or 99% identity to the amino acid sequence set forth in SEQ ID NO:29; or
(iii) comprises an amino acid sequence having at least 85%, 90%, 91%, 92%, 93%, 94%, 95%, 96%, 97%, 98% or 99% identity to the amino acid sequence set forth in SEQ ID NO:21 and having an S228P deletion mutation.

12. The anti-OX40L antibody or antigen-binding fragment according to any one of claims 1 to 11, which comprises a light chain constant region, said light chain constant region being a lambda or kappa light chain constant region, preferably, said light chain constant region
(i) comprises or consists of an amino acid sequence having at least 85%, 90%, 91%, 92%, 93%, 94%, 95%, 96%, 97%, 98% or 99% identity to the amino acid sequence of SEQ ID NO:22 said amino acid sequence; or
(ii) comprises or consists of the amino acid sequence of SEQ ID NO:22.

13. The anti-OX40L antibody or antigen-binding fragment according to any one of claims 1 to 12, which comprises a heavy chain, wherein said heavy chain
(i) comprises an amino acid sequence having at least 85%, 90%, 91%, 92%, 93%, 94%, 95%, 96%, 97%, 98% or 99% identity with an amino acid sequence selected from SEQ ID NO: 9 or 19 or consists of said amino acid sequence; or
(ii) comprises an amino acid sequence selected from SEQ ID NO: 9 or 19 or consists of said amino acid sequence; or
which comprises a light chain, wherein said light chain
(i) comprises an amino acid sequence having at least 85%, 90%, 91%, 92%, 93%, 94%, 95%, 96%, 97%, 98% or 99% identity with an amino acid sequence selected from SEQ ID NO: 10 or 20 or consists of said amino acid sequence; or
(ii) comprises an amino acid sequence selected from SEQ ID NO: 10 or 20 or consists of said amino acid sequence.

14. An anti-OX40L antibody or an antigen-binding fragment thereof, comprising a heavy chain and
a light chain, wherein
(i) the heavy chain comprises or consists of an amino acid sequence having at least 85%, 90%, 91%, 92%, 93%, 94%, 95%, 96%, 97%, 98% or 99% identity with the amino acid sequence set forth in SEQ ID NO:9, and the light chain comprises or consists of an amino acid sequence having at least 85%, 90%, 91%, 92%, 93%, 94%, 95%, 96%, 97%, 98% or 99% identity with the amino acid sequence set forth in SEQ ID NO:10;
(ii) the heavy chain comprises or consists of the amino acid sequence of SEQ ID NO:9, and the light chain comprises or consists of the amino acid sequence of SEQ ID NO:10;
(iii) the heavy chain comprises or consists of an amino acid sequence having at least 85%, 90%, 91%, 92%, 93%, 94%, 95%, 96%, 97%, 98% or 99% identity with the amino acid sequence set forth in SEQ ID NO:19, and the light chain comprises or consists of an amino acid sequence having at least 85%, 90%, 91%, 92%, 93%, 94%, 95%, 96%, 97%, 98% or 99% identity with the amino acid sequence set forth in SEQ ID NO:20; or
(iv) the heavy chain comprises or consists of the amino acid sequence of SEQ ID NO: 19, and the light chain comprises or consists of the amino acid sequence of SEQ ID NO:20.

15. The anti-OX40L antibody or antigen-binding fragment according to any one of claims 1 to 14, which comprises a heavy chain and a light chain, wherein
(i) the heavy chain comprises or consists of the amino acid sequence of SEQ ID NO:9, and the light chain comprises or consists of the amino acid sequence of SEQ ID NO:10; or
(ii) the heavy chain comprises or consists of the amino acid sequence of SEQ ID NO:19, and the light chain comprises or consists of the amino acid sequence of SEQ ID NO:20.

16. The anti-OX40L antibody or antigen-binding fragment according to any one of claims 1 to 15, wherein said antibody is a monoclonal antibody.

17. The anti-OX40L antibody or antigen-binding fragment according to any one of claims 1 to 16, wherein said antibody is a humanized antibody or a chimeric antibody.

18. The anti-OX40L antibody or antigen-binding fragment according to any one of claims 1 to 17, wherein said antigen-binding fragment is an antibody fragment selected from the group consisting of Fab, Fab', Fab'-SH, Fv, single-chain antibody (e.g., scFv), (Fab')₂, single-domain antibody such as VHH, dAb (domain antibody), bivalent antibody, or linear antibody.

19. The anti-OX40L antibody or antigen-binding fragment according to any one of claims 1 to 18, wherein said antibody or antigen-binding fragment has one or more of the following properties:
a) binds to OX40L (e.g., human OX40L) with high affinity;
b) effectively blocks the interaction between OX40L and its receptor OX40 at both protein and cellular levels;
c) inhibits the OX40/OX40L signaling pathway;
d) inhibits the activation, proliferation, and/or differentiation of T cells;
e) reduces the activation of T cells and/or the production of inflammatory factors;
f) treats graft-versus-host disease.

20. An isolated nucleic acid encoding the anti-OX40L antibody or antigen-binding fragment according to any one of claims 1 to 19.

21. A vector comprising the nucleic acid of claim 20, preferably said vector is an expression vector.

22. A host cell comprising the nucleic acid of claim 20 or the vector of claim 21, preferably said host cell is prokaryotic or eukaryotic, more preferably selected from yeast cells, mammalian cells (e.g., 293 cells or CHO cells, e.g., CHO-K cells or HEK293 cells), or other cells suitable for the preparation of antibodies or antigen-binding fragments.

23. A method for preparing an anti-OX40L antibody or an antigen-binding fragment thereof, said method comprising
a) culturing the host cell of claim 22 under conditions suitable for the expression of the nucleic acid encoding the anti-OX40L antibody or antigen-binding fragment thereof according to any one of claims 1 to 19,
b) optionally isolating said antibody or antigen-binding fragment thereof,
c) optionally said method also includes recovering said anti-OX40L antibody or antigen-binding fragment thereof from said host cell, optionally, said antibody is purified, e.g., by Protein A purification.

24. An immunoconjugate comprising the anti-OX40L antibody or antigen-binding fragment according to any one of claims 1 to 19 and another agent, such as a toxin, small molecule drug, cytotoxic agent, cellular apoptotic agent, chelating agent, immune modulator, such as an anti-inflammatory agent or immunosuppressive agent.

25. A pharmaceutical composition comprising the anti-OX40L antibody or antigen-binding fragment according to any one of claims 1 to 19 or the immunoconjugate of claim 24, and optionally a pharmaceutically acceptable excipient.

26. A pharmaceutical combination comprising the anti-OX40L antibody or antigen-binding fragment according to any one of claims 1 to 19 or the immunoconjugate of claim 24, and one or more other therapeutic agents, such as said therapeutic agent is selected from the group consisting of cytokines, other antibodies, small molecule drugs, or immune modulators (e.g., immunosuppressive agents).

27. A method for preventing or treating a disease or condition that are associated with abnormal activation of OX40L/OX40-mediated pathways in an individual, said method comprising administering to said subject an effective amount of the anti-OX40L antibody or antigen-binding fragment according to any one of claims 1 to 19, or the immunoconjugate of claim 24, or the pharmaceutical composition of claim 25, or the pharmaceutical combination of claim 26.

28. The method of claim 27, wherein said subject has abnormal activation of the OX40L/OX40-mediated signaling pathway compared to a healthy individual.

29. The method of claim 27 or 28, wherein said disease or condition is selected from autoimmune diseases or graft-versus-host disease, for example, the autoimmune diseases include, but are not limited to, allergic dermatitis, asthma, systemic lupus erythematosus, Sjogren's syndrome, immune thrombocytopenic purpura, multiple sclerosis, lupus nephritis, amyotrophic lateral sclerosis, rheumatoid arthritis, non-rheumatoid arthritis, contact dermatitis, hyper IgE syndrome, inflammatory bowel disease, myasthenia gravis, Graves' disease, hemolytic anemia, psoriasis, atopic dermatitis, allergic asthma, or idiopathic inflammatory disease.

30. The method according to any one of claims 27 to 29, wherein said method further comprises administering one or more other therapies, such as therapeutic modalities and/or other therapeutic agents, such as said therapeutic agent is selected from the group consisting of cytokines, other antibodies, small molecule drugs, or immune modulators (e.g., immunosuppressive agents).
